# EUROPEAN PATENT APPLICATION

(11) **EP 4 295 877 A1**
(43) Date of publication of application: **27.12.2023**
(21) Application number: 22778524.3
(22) Date of filing: 11.03.2022
(51) Int. Cl.: A61M 15/00, A61M 31/00

(54) **INHALATION DEVICE**

(30) Priority: 30.03.2021 CN 202110340115; 30.03.2021 CN 202120665154 U; 30.03.2021 CN 202120648087 U
(71) Applicant: CF Pharmtech, Inc., Jiangsu 215143 (CN)
(72) Inventor: LI, Qi, Suzhou, Jiangsu 215143 (CN); XU, Wanling, Suzhou, Jiangsu 215143 (CN); YIN, Jiehong, Suzhou, Jiangsu 215143 (CN); ALAI, Shailaja Milind, Suzhou, Jiangsu 215143 (CN)
(74) Representative: Canzler & Bergmeier Patentanwälte Partnerschaft mbB
(86) International application number: PCT/CN2022/080370
(87) International publication number: WO 2022/206340

(57) **Abstract**

Disclosed is an inhalation device, which, by means of a simplified structure, reduces the manufacturing difficulty and cost, and ensures the use effect. In the inhalation device, a filter member closely fits to a capsule placement opening, and when a mouthpiece is clamped to a cover plate, a mesh portion is integrally located in a capsule compartment. Therefore, when a medicinal powder is inhaled, airflow can better pass through the mesh portion under the action of a negative pressure to flow in an ideal flow direction, thereby ensuring the medicinal powder to be effectively inhaled.

## Description

### TECHNICAL FIELD

The present application relates to the technical field of medical apparatuses and instruments, in particular, to an inhalation device.

### BACKGROUND

In current drug administration methods, drugs can be taken by direct inhalation for some respiratory diseases. For example, at the time of drug administration, a capsule containing pharmaceutical powder is pierced using a special drug administration device, and the pharmaceutical powder is inhaled into the respiratory tract. However, in the prior art, common drug administration devices are usually complex in structure, thereby increasing the manufacturing cost.

### SUMMARY OF THE INVENTION

In view of the disadvantages of the above related technology, an object of the present application is to provide an inhalation device to overcome the technical problems of complex structure and high manufacturing cost of the drug administration devices in the above related technology.

In order to achieve the above and other related objects, provided in the present application is an inhalation device, comprising: a mouthpiece, which comprises a mouthpiece body and an inhalation channel formed in the mouthpiece body, the bottom of the inhalation channel is of a tubular structure; a filter member, which comprises a sleeving portion sleeved on a bottom end of the inhalation channel and a mesh portion integrally formed with the sleeving portion, to avoid that capsule fragments generated after a capsule is pierced enter the inhalation channel; a capsule chamber, which is located below the mouthpiece and comprises a deck and a compartment integrally formed on a lower side of the deck and configured to hold a capsule in a use state, the deck is provided with a capsule insertion port which is in communication with the compartment and is correspondingly engaged with the mesh portion; a piercing assembly, which is arranged on one side of the capsule chamber and comprises a button member and piercing needles which are movably arranged on one side of the capsule chamber, the button member, when stressed in a use state, moves relative to the capsule chamber to drive the piercing needles to pierce the capsule in the capsule chamber; a base, which comprises a base body for accommodating the compartment and part of the piercing assembly; and a cap body, which covers the mouthpiece when in a state of being snap-fitted on the base; in a state in which the capsule is in the capsule chamber and the capsule is pierced, when a negative pressure is generated in the inhalation channel, pharmaceutical powder in the capsule enters the inhalation channel via the mesh portion through the capsule insertion port.

In some embodiments of the present application, the cap body, the mouthpiece, the capsule chamber and the base are coaxially hinged on distal sides thereof by means of hinge portions.

In some embodiments of the present application, the cap body comprises a first snap-fit portion located on a proximal side of the cap body and configured to be snap-fitted on an edge of a proximal side of the deck.

In some embodiments of the present application, the first snap-fit portion is a bump located on an inner side of a proximal end of the cap body, and the deck is provided at a proximal end thereof with an end surface that matches the bump, so that the cap body and the deck are snap-fitted on proximal sides thereof.

In some embodiments of the present application, the cap body comprises a first anti-skid portion located on the proximal side of the cap body.

In some embodiments of the present application, the mouthpiece body comprises:
a mounting portion configured to be snap-fitted on the deck of the capsule chamber; and a sucking portion integrally formed with the mounting portion and having an approximately flat elliptical structure adapting to the mouth of a human body; a transition surface is provided between the mounting portion and the sucking portion.

In some embodiments of the present application, the mouthpiece comprises second snap-fit portions located on a proximal side of the mouthpiece, and the deck comprises third snap-fit portions located on the proximal side of the deck, the second snap-fit portions and the third snap-fit portions being snap-fitted at proximal ends thereof.

In some embodiments of the present application, the second snap-fit portions are hook structures formed on a lower surface of the mouthpiece, and the third snap-fit portions are recesses formed on the deck and corresponding to the hook structures.

In some embodiments of the present application, the mouthpiece comprises a second anti-skid portion located on the proximal side of the mouthpiece.

In some embodiments of the present application, the bottom end of the inhalation channel extends out of the mouthpiece body, so that when the mouthpiece is snap-fitted on the deck, the filter member sleeved on the inhalation channel presses the capsule insertion port, so as to form a closed channel of airflow.

In some embodiments of the present application, contact surfaces of the filter member and the capsule chamber are provided with matching end surfaces so that the filter member closely fits the capsule chamber when the mouthpiece is snap-fitted on the capsule chamber.

In some embodiments of the present application, the deck comprises side wings provided with fourth snap-fit portions, which are configured to be snap-fitted on fifth snap-fit portions on an edge of an upper side of the base.

In some embodiments of the present application, the deck is provided with a first air inlet, which is configured to facilitate the entry of outside air into an inner space of the base by means of the first air inlet when a negative pressure is generated in the inhalation channel.

In some embodiments of the present application, the compartment of the capsule chamber comprises a main compartment having a diameter greater than a transverse cross-sectional diameter of the capsule and smaller than a longitudinal cross-sectional diameter of the capsule.

In some embodiments of the present application, an auxiliary compartment in communication with the main compartment is provided at the bottom of the compartment of the capsule chamber, the auxiliary compartment is provided with a second air inlet at a bottom end thereof, a gap is disposed between the second air inlet and a bottom surface of the base, and the auxiliary compartment has a diameter smaller than the transverse cross-sectional diameter of the capsule.

In some embodiments of the present application, the compartment of the capsule chamber comprises a mounting structure, which is configured to match the piercing assembly so that the piercing assembly pierces a capsule in the capsule chamber by using the piercing needles thereof during movement relative to the capsule chamber.

In some embodiments of the present application, the mounting structure comprises: a mounting plate provided with symmetrical slots; an abutment portion formed on the mounting plate and located between the symmetrical slots; and two tube portions formed on the mounting plate, respectively located on upper and lower sides of the abutment portion, in communication with the compartment and configured to limit piercing directions of the piercing needles.

In some embodiments of the present application, the piercing assembly further comprises a reset mechanism located between the button member and the abutment portion and configured to be pressed, when the button member moves toward the capsule chamber, to provide a reset force to restore the piercing needles and the button member to initial positions thereof.

In some embodiments of the present application, the piercing needles are arranged in the tube portions in a penetrating manner, and proximal ends of the piercing needles are fixedly connected to the button member.

In some embodiments of the present application, the button member comprises a pressing portion and movable connecting portions integrally formed with the pressing portion, where a limiting portion matching one of the symmetrical slots is provided at a distal end of each of the movable connecting portions.

In some embodiments of the present application, a third anti-skid portion is provided on an outer surface of the pressing portion.

In some embodiments of the present application, a needle tip portion of each of the piercing needles has an inclined surface.

In some embodiments of the present application, two piercing needles are provided and are arranged one on top of the other, and a distance between the two piercing needles arranged one on top of the other is less than a height of the capsule and greater than one half of the height of the capsule.

In some embodiments of the present application, the base body of the base comprises a fourth anti-skid portion located on a distal side of the base body.

In some embodiments of the present application, the base is provided, at a proximal end thereof, with an opening, which is configured to provide a movement space for the piercing assembly.

In some embodiments of the present application, the base body of the base is provided with a second stop portion, which is configured to limit a movement position of the piercing assembly relative to the capsule chamber.

In some embodiments of the present application, the base further comprises a transparent inspection window mounted on the base body, and the compartment of the capsule chamber has a transparent wall surface, so as to observe the state of the capsule in the capsule chamber through the base in a use state.

In some embodiments of the present application, the transparent inspection window is U-shaped, and correspondingly, a mounting groove extending to front and back sides of the base body is formed in the bottom surface of the base body of the base, so as to mount the transparent inspection window.

In some embodiments of the present application, the base is made of a transparent material, and the compartment of the capsule chamber is provided with a transparent wall surface, so as to observe the state of the capsule in the capsule chamber through the base in a use state.

In summary, by means of a simplified structure of the inhalation device in the present application, the difficulty and cost of production are reduced while the use effect is ensured. In the inhalation device of the present application, the filter member closely fits the capsule insertion port, and when the mouthpiece is snap-fitted on the deck, the mesh portion is integrally located in the capsule chamber. Therefore, when pharmaceutical powder is inhaled, airflow can better pass through the mesh portion under the action of a negative pressure to flow in an ideal flow direction, thereby ensuring effective inhalation of the pharmaceutical powder.

Furthermore, matching guide structures of the deck and the button member can avoid the deviation of the piercing needles during piercing or the generation of a sense of resistance caused by the unstable movement position of the button member during pressing. The cap body, the mouthpiece, the filter member and the base of the inhalation device are coaxially connected and anti-skid structures are provided at a plurality of positions, which conform to the ergonomic design.

Other aspects and advantages of the present application will be readily apparent to a person skilled in the art from the detailed description below. Only exemplary embodiments of the present application are shown and described in the following detailed description. As will be recognized by a person skilled in the art, the content of the present application enables a person skilled in the art to make modifications to the specific embodiments disclosed without departing from the spirit and scope of the present application involved in the present application. Accordingly, the accompanying drawings of the present application and the description in the specification are illustrative only and are not intended to be limiting.

### BRIEF DESCRIPTION OF DRAWINGS

The specific features of the invention involved in the present application are as shown in the appended claims. The features and advantages of the invention involved in the present application can be better understood by reference to the exemplary embodiments and accompanying drawings described in detail hereinafter. The accompanying drawings are briefly described as follows:
FIG. 1a shows an exploded schematic view of the structure of an inhalation device of the present application in one embodiment;
FIG. 1b shows a schematic diagram of the hinge relationship of a cap body, a mouthpiece, a capsule chamber and a base of the present application in one embodiment;
FIG. 2 shows a schematic structure diagram of the mouthpiece of the present application in one embodiment;
FIG. 3 shows a schematic structure diagram of a filter member of the present application in one embodiment;
FIG. 4 shows a schematic structure diagram of the cap body of the present application in one embodiment;
FIG. 5 shows a schematic structure diagram of the capsule chamber of the present application in one embodiment;
FIG. 6 shows a schematic structure diagram of the cap body and the capsule chamber of the present application in one embodiment;
FIG. 7 shows a schematic structure diagram of the cap body of the present application in another embodiment;
FIG. 8 shows a schematic diagram of the deck of the present application in another embodiment;
FIG. 9 shows a schematic diagram of the mouthpiece and the capsule chamber, which are snap-fitted, of the present application in one embodiment;
FIG. 10 shows a schematic structure diagram of the filter member and the capsule chamber of the present application in one embodiment;
FIG. 11 shows a schematic structure diagram of the filter member of the present application in another embodiment;
FIG. 12a shows a schematic structure diagram of the filter member and the mouthpiece of the present application in one embodiment;
FIG. 12b shows a cross-sectional view of the filter member and the mouthpiece of the present application in one embodiment;
FIG. 13 shows a schematic structure diagram of the filter member of the present application in one embodiment;
FIG. 14a shows a schematic structure diagram of the base of the present application in one embodiment;
FIG. 14b shows an enlarged partial structure diagram of part A in FIG 14a of the present application;
FIG. 15 shows a schematic structure diagram of the capsule chamber of the present application in one embodiment;
FIG. 16 shows a structural schematic diagram of a piercing assembly and the capsule chamber of the present application in yet another embodiment;
FIG. 17 shows a structural schematic diagram of the capsule chamber and a capsule of the present application in one embodiment;
FIG. 18 shows a schematic structure diagram of piercing needles of the present application in one embodiment;
FIG. 19 shows a schematic structure diagram of a button member of the present application in one embodiment;
FIG. 20a shows a schematic structure diagram of the button member of the present application in another embodiment;
FIG. 20b is an enlarged schematic view of part G in FIG 20a;
FIG. 21 shows a schematic structure diagram of the piercing assembly of the present application in one embodiment;
FIG. 22 shows a schematic structure diagram of the button member of the present application in one embodiment;
FIG. 23 shows a schematic exploded structure diagram of the piercing assembly and the capsule chamber of the present application in another embodiment;
FIG. 24 shows a schematic structure diagram of the base of the present application in another embodiment;
FIG. 25 shows a schematic structure diagram of the base of the present application in yet another embodiment;
FIG. 26 shows a schematic structure diagram of the capsule of the present application in one embodiment;
FIG. 27 shows a schematic structure diagram of the inhalation device of the present application in yet another embodiment; and
FIG. 28 shows a schematic diagram of the airflow direction of the inhalation device of the present application in one embodiment.

### DETAILED DESCRIPTION OF THE INVENTION

The embodiments of the present application are described hereinafter using specific embodiments, and other advantages and effects of the present application will be readily understood by those familiar with the art from the disclosure of this specification.

In the following description, reference is made to the accompanying drawings which describe several embodiments of the present application. It should be understood that other embodiments may also be used, and changes in module or unit composition and electrical and operational changes may be made without departing from the spirit and scope of the present disclosure. The following detailed description should not be considered as limiting, and the scope of the embodiments of the present application is defined only by the claims of the patent as published. The terms used herein are merely intended to describe specific embodiments rather than to limit the present application.

Although in some examples, the terms "first", "second", etc. are used herein to describe various elements, information or parameters, such elements or parameters should not be limited by these terms. These terms are merely used to distinguish one element or parameter from another. For example, a first snap-fit portion may be referred to as a second snap-fit portion, and similarly, a second snap-fit portion may be referred to as a first snap-fit portion, without departing from the scope of the various described embodiments. Both a first snap-fit portion and a second snap-fit portion describe a snap-fit portion. Unless it is clearly indicated in the context, otherwise, they are not the same snap-fit portion. Similar cases also include a first stop portion and a second stop portion, or a first anti-skid portion and a second anti-skid portion.

Furthermore, as used herein, the singular forms "a/an", "one" and "the" are intended to include plural forms as well, unless the contrary is indicated in the context. It should be further understood that the terms "contain" and "comprise" indicate the presence of the described features, steps, operations, elements, components, items, categories, and/or groups, but do not rule out the presence, occurrence, or addition of one or more other features, steps, operations, elements, components, items, categories, and/or groups. The terms "or" and "and/or" as used herein are construed to be inclusive or mean any one or any combination. Thus, "A, B or C" or "A, B and/or C" means "any of the following: A; B; C; A and B; A and C; B and C; and A, B and C". Exceptions to this definition occur only when combinations of elements, functions, steps, or operations are inherently mutually exclusive in certain ways.

As described in the background art, in some embodiments, drug administration devices for treating respiratory diseases are generally complex in structure. For example, in order to avoid capsule fragments being inhaled together during inhalation of pharmaceutical powder after a capsule is pierced, a drug administration device is provided with a metal mesh, and in order to fix the metal mesh onto a mouthpiece, a fixed frame having a special structure needs to be separately manufactured to embed the metal mesh into the fixed frame, and the manufacture and assembly of different materials result in an increase in complexity of manufacture and production and cost of materials; moreover, the mesh of a metal material tends to deform when used improperly, the deformation of the mesh directly affects the direction of airflow during use, and the metal material, exposed in a wet environment for a long time during its use, also oxidizes or rusts easily, which therefore also shortens the product life.

Furthermore, in some related technologies, a capsule chamber and a capsule chamber deck of an inhaler serving as a drug administration device are two independent elements, which are combined to form a compartment for holding a capsule. However, the two independent elements need to be combined in the assembly process, which increases material and production costs; and more importantly, although the two independent elements, the capsule chamber and the capsule chamber deck, are fixedly mounted, with the growth of time of use by a patient or the increase of times of opening and closing, a gap may appear between the capsule chamber and the capsule chamber deck, which affects the airflow in use such that the airflow during drug inhalation cannot achieves a desirable effect, thus reducing the efficiency of inhalation. Hence, the inhalation efficiency of a drug administration device will have a significant impact on both the efficiency and effect of drug administration.

In addition, a piercing needle of a drug administration device for treating respiratory diseases is usually driven by a button member to pierce a capsule in a capsule chamber. However, in the process of pressing the button member, there will be cases where the stroke is unstable, and the piercing needle will fluctuate up and down to a certain extent when the button member is being pressed. As a result, on one hand, a force is not applied smoothly during pressing, and on the other hand, the piercing needle is unable to pierce the capsule linearly. If the capsule is pierced at a wrong position, capsule fragments are liable to be produced, and a powdered drug cannot be effectively released in the inhalation process. Moreover, since a patient cannot sense the position of the piercing needle when using the inhalation device, excessive puncture is likely to occur in the process of pressing the button member, generating more capsule fragments. In some embodiments, even if the position of the piercing needle can be clearly seen, there will also be a problem of excessive puncture because the magnitude of force for pressing the button member cannot be accurately controlled.

In view of this, provided in the present application is an inhalation device which, in some embodiments, is a drug administration device facilitating the inhalation of pharmaceutical powder by a patient by respiratory inhalation from a capsule encapsulated with a powdered drug. In some applications, the inhalation device serving as a drug administration device is also referred to as an inhaler, a pharmaceutical powder inhaler, a dry powder inhaler, or the like. During use, a patient can load the capsule into a capsule chamber located in the inhaler before use, and then the patient can press a button which can drive a piercing needle, causing the piercing needle to switch from a static state to a moving state to pierce the capsule in the capsule chamber, so that the capsule releases a powdered drug therein under the action of a negative pressure. Examples of the powdered drug include, but are not limited to, tiotropium bromide, indacaterol and other drugs that can be administered by respiratory inhalation.

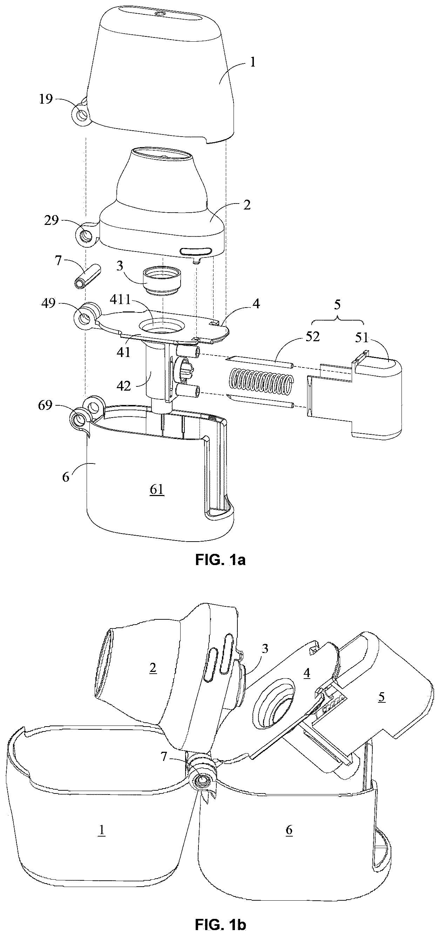

In one exemplary embodiment, please refer to FIG. 1a, which shows an exploded schematic view of the structure of the inhalation device of the present application in one embodiment. As shown in the figure, the inhalation device comprises a cap body 1, a mouthpiece 2, a filter member 3, a capsule chamber 4, a piercing assembly 5, and a base 6.

The cap body is a component configured to cover the mouthpiece 2 in a state of being snap-fitted on the base 6, so as to prevent the mouthpiece from being contaminated by the outside.

In the present application, the snap-fit represents a physical manner of fit between two components by means of which the two components can be quickly or easily fixed to or separated from each other using mechanical deformation (e.g., interference fit) or mechanical interference, examples in the embodiments including a manner in which a hook fits a slot, a manner in which a hook fits a hook, a manner in which a protrusion fits a groove, a manner in which a protrusion fits a protrusion, etc. In the present application, a snap-fit structure between two components will be described herein on the basis of the mechanical structures of different components in their respective states of implementation in combination with figures.

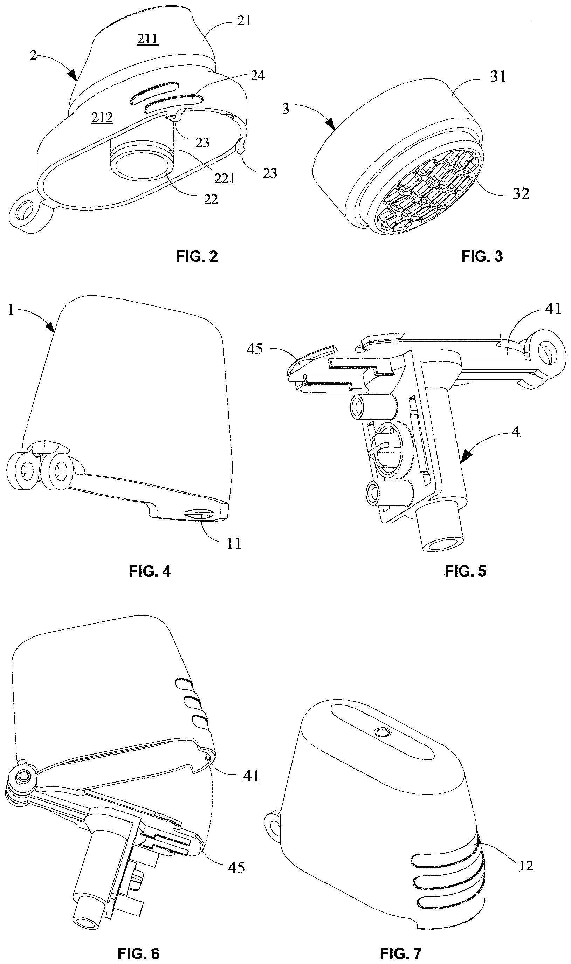

The mouthpiece is a component configured to match the mouth of a human body to inhale the pharmaceutical powder in a capsule in the capsule chamber into the human body. In this embodiment, please refer to FIG. 2, which shows the structure of the mouthpiece of the present application in one embodiment, an inhalation channel 22 is integrally formed in a mouthpiece body 21.

In one implementation, in order to facilitate the formation of a smooth airflow channel after the inhalation channel is engaged with the capsule chamber, the bottom of the inhalation channel is of a tubular structure. The tubular structure may be a round tube, a square tube or a tube of any other shape as long as it allows a powdered drug to pass through. However, in this embodiment, a round tube, namely, a tubular structure with a round cross section, is used in consideration of the inhalation efficiency.

In the present application, the integrally formed structure represents a structure in which multiple components having respective functions are integrated into a whole and cannot be physically detached by non-destructive means, and the components are usually integrated as a whole at the manufacture stage, such as, being injection molded using a mold, or being integrally formed in an additive manufacturing (3D printing) manner.

The filter member represents a component functioning to filter the object in the capsule chamber and being beneficial to generating a high-efficiency inhalation airflow. In some embodiments, the filter member can be sleeved on the inhalation channel at the bottom.

In the present application, the sleeving means that there is a difference in diameter between two components so that the component having a larger diameter can be sleeved on the component having a smaller diameter. Thus, on one hand, the filter member needs to function as a filter to allow only the pharmaceutical powder to pass through the inhalation channel, and on the other hand, the filter member needs to be fixed to the bottom of the inhalation channel 22.

In one embodiment, please refer to FIG. 3, which shows a schematic structure diagram of the filter member of the present application in one embodiment. As shown in the figure, the filter member 3 comprises a sleeving portion 31 and a mesh portion 32, the mesh portion is configured to avoid that capsule fragments generated after a capsule is pierced enter the inhalation channel, and the sleeving portion is configured to be sleeved on a bottom end of the inhalation channel 22 having a tubular structure. Moreover, in the embodiment, the mesh portion 32 and the sleeving portion 31 are of an integrally formed structure, so that manufacture and production are facilitated while filtering and fixing functions are achieved, the step of assembling the mesh portion and the sleeving portion is omitted, the stability of a mechanical fit by means of which the filter member comprising the mesh portion and the sleeving portion is engaged with the inhalation channel is ensured, and the material management cost is also saved because of a simplified structure. In addition, because the mesh portion 32 and the sleeving portion 31 are of an integrally formed structure and are designed using the same material, deformation will not occur between the mesh portion 32 and the sleeving portion 31 with the increase in time of use by a patient. The structural stability of the mesh portion 32 ensures that matching contact surfaces are formed after the mesh portion 32 is engaged with the capsule chamber, to facilitate the formation of a smooth airflow channel, thereby ensuring that the airflow when a patient inhales, especially when inhaling a drug, can guarantee the inhalation efficiency, thereby ensuring the drug administration efficiency and drug administration effect. In addition, in the embodiment, a capsule insertion port 411 on a deck is correspondingly engaged with the mesh portion 32 so that the mesh portion 32 closely fits the capsule insertion port 411 so as to ensure the inhalation efficiency.

The capsule chamber is a component configured to hold a capsule and maintain a connection with the piercing assembly, the mouthpiece and the base. Please refer further to FIG. 1a, the capsule chamber 4 is located below the mouthpiece 2, and the capsule chamber comprises a deck 41 and a compartment 42 located below the deck 41 and configured to hold a capsule. In the embodiment, the capsule is substantially in an upright state when placed in the compartment 42, namely, one end of the capsule is located at the bottom of the compartment 42, and the other end of the capsule is adjacent to the top of the compartment 42.

The deck and the compartment may be integrally formed, and may also be independent of each other and keep a fixed state after assembly. In one implementation, in the case where the deck and the compartment are integrally formed, the integral forming between the deck and the compartment makes the deck and the compartment seamlessly connected, the manufacturing cost and difficulty are reduced, and the step of assembling the deck and the compartment is omitted. It should be emphasized that in the embodiment, as the deck 41 and the compartment 42 of the capsule chamber are of an integrated structure, the material and production costs are reduced, and more importantly, even with the increase in time of use by a patient or the increase of times of opening and closing, there will be no gap between the capsule chamber and the capsule chamber deck, thus ensuring that the airflow when a patient inhales, especially when inhaling a drug, can guarantee the inhalation efficiency, thereby ensuring the drug administration efficiency and drug administration effect.

The deck 41 is located above the compartment 42, and the deck 41 is provided with a capsule insertion port 411. On one hand, the capsule insertion port 411 is in communication with the compartment 42, and in a use state, a patient places a capsule in the compartment 42 via the capsule insertion port 411.

In the present application, the communication refers to spatial communication so that airflow or pharmaceutical powder carried by airflow can pass through, and in some expressions, the communication is also used interchangeably with connection, both referring to spatial connection of two physical spaces.

On the other hand, the capsule insertion port 411 is correspondingly engaged with the mesh portion 32 so that the capsule insertion port closely fits the mesh portion so as to ensure the inhalation efficiency. The deck 41 and the compartment 42 are integrally formed so that production and manufacture are facilitated and the step of assembling the deck 41 and the compartment 42 is omitted. It should be noted here that the capsule is not necessarily part of the inhalation device, and in some cases, in order to avoid the powder in the capsule getting damp, only in a use state is the capsule taken out and put into the capsule chamber so as to inhale the pharmaceutical powder in the capsule.

The piercing assembly is a component configured to pierce the capsule in the capsule chamber. The piercing assembly 5 is located on one side of the capsule chamber 4. The piercing assembly 5 comprises a button member 51 and piercing needles 52. The button member 51 can move relative to the capsule chamber. The compartment of the capsule chamber 4 is provided with channels for the piercing needles to pass through. In a use state, when a pressing force is applied to the button member, the button member moves relative to the capsule chamber and drives the piercing needles 52 to pierce the capsule in the compartment 42. In the present application, relative movement refers to the movement of multiple components close to or away from each other. Hence, the button member being movable relative to the capsule chamber here means that the button member can move close to or away from the capsule chamber.

The base is a component which is configured to accommodate the capsule chamber and serve as part of an airflow channel, and is dust-proof and convenient for a user to hold and operate. The base 6 comprises a base body 61, which has a hollow structure inside and is configured to accommodate the compartment 42 of the capsule chamber and part of the piercing assembly 5.

When the piercing needles pierce a capsule (or break the wall of the capsule), the consistency and stability of the piercing directions are particularly important for the inhalation device. On the basis of this, in the embodiments of the present application, a first guide portion is provided on a lower surface of a side of the deck corresponding to the button member, i.e., a proximal side of the deck, and the button member is provided with a second guide portion matching the first guide portion, so that when the button member moves towards the capsule chamber, in the process of pushing a button to pierce a capsule, two needles can be guided to move stably on a horizontal line, so as to ensure that holes in a capsule shell are kept relatively consistent in shape and size after the capsule is pierced by the needles. Therefore, not only can pharmaceutical powder in the capsule chamber escape smoothly under the action of a negative pressure, but also in the process of piercing the capsule, fragments of capsule skin will not be produced due to the shaking of the needles.

According to the mode of use in the embodiment, first, the cap body 1 and the mouthpiece 2 are opened, a capsule is placed in the capsule insertion port 411 of the capsule chamber 4, and the capsule enters the compartment 42 from the capsule insertion port 411; then, the mouthpiece 2 is placed on the deck 41 of the capsule chamber 4 to enable the inhalation channel 22 to be communicated with the capsule insertion port 411; next, the button member 51 drives the piercing needles 52 to pierce the capsule located in the compartment 42 so as to form a pharmaceutical powder outlet in the capsule; and at the moment, a user sucks on an upper portion of the mouthpiece with his/her mouth and inhales, such that a negative pressure is generated in the inhalation channel, and the pharmaceutical powder, because of its small diameter, can leave the capsule via the pharmaceutical powder outlet of the capsule under the action of the negative pressure, pass through the filter member 3, and enter the inhalation channel 22 so as to be inhaled into the body of the user, and capsule fragments that may be generated are blocked by the filter member 3 and will not enter the inhalation channel.

In the embodiment, as the mesh portion 32 and the sleeving portion 31 are of an integrally formed structure, manufacture and production are facilitated while filtering and fixing functions are achieved, the step of assembling the mesh portion and the sleeving portion is omitted, the stability of a mechanical fit by means of which the filter member comprising the mesh portion and the sleeving portion is engaged with the inhalation channel is ensured, and the material management cost is also saved because of a simplified structure. Moreover, because the mesh portion 32 and the sleeving portion 31 are of an integrally formed structure and are designed using the same material, deformation will not occur between the mesh portion 32 and the sleeving portion 31 with the increase in time of use by a patient. The structural stability of the mesh portion 32 ensures that matching contact surfaces are formed after the mesh portion 32 is engaged with the capsule chamber, to facilitate the formation of a smooth airflow channel, thereby ensuring that the airflow when a patient inhales, especially when inhaling a drug, can guarantee the inhalation efficiency, thereby ensuring the drug administration efficiency and drug administration effect.

It should be understood that the terms "proximal" and "distal" used in the present application are defined with respect to the piercing assembly. Therefore, in various embodiments of the present application, for ease of description, a side of the inhalation device that is close to the piercing assembly is defined as a proximal side, and a side thereof that is away from the piercing assembly is defined as a distal side, that is, a side with numbering of 7, 19, 29, 49 and 69 shown in FIG. 1a of the present application is a distal side, and an opposite side is a proximal side. In addition, the terms "front" and "back" used in the present application are defined with respect to the illustrated viewing direction for ease of explanation. Specifically, "front" in the present application is defined as facing, with the direction in which the piercing assembly is located as a reference, a left side of the direction in which a shaft 7 is located, i.e. a side in FIG. 1a closer to the illustrated viewing direction; and "back" in the present application is defined as facing, with the direction in which the piercing assembly is located as a reference, a right side of the direction in which the shaft 7 is located, i.e. a side in FIG. 1a away from the illustrated viewing direction. It should also be understood that for ease of description, spatial terms such as "top surface", "bottom surface", etc. are used in the present application in the state of placement of the inhalation device as shown in the figures. However, these spatial terms are not limiting or absolute because surgical medical instruments can be used in multiple directions and positions in various use scenarios.

In one exemplary embodiment, for ease of use, the cap body, the mouthpiece, the capsule chamber and the base are coaxially hinged on distal sides thereof by means of hinge portions.

In a possible implementation, please refer further to FIG. 1a, hinge portions (19, 29, 49, 69) are provided on distal sides of the cap body 1, the mouthpiece 2, the capsule chamber 4, and the base 6. The cap body 1, the nozzle 2, the capsule chamber 4 and the base 6 are coaxially hinged by means of a shaft 7 passing through their respective hinge portions. As shown in FIG. 1a, the cap body 1, the mouthpiece 2, the capsule chamber 4, and the base 6 are hinged by means of one shaft 7 in such a manner that the shaft 7 passes through their respective hinge portions. In a use state, the state in which the cap body 1, the mouthpiece 2, the capsule chamber 4 and the base 6 are hinged is shown in FIG. 1b, which shows a schematic diagram of the hinge relationship of the cap body, the mouthpiece, the capsule chamber and the base of the present application in one embodiment.

In one exemplary embodiment, in order to prevent the cap body from loosening and not being able to protect the mouthpiece in a non-use state, matching snap-fit structures are arranged between the cap body and the deck, so that the cap body is opened to expose the mouthpiece by means of the snap-fit structures during use, and the cap body is closed to cover the mouthpiece in a non-use state.

In one embodiment, a structure matching a first snap-fit portion is provided on a proximal side of the deck. The specific structure of the first snap-fit portion may be designed according to the manner in which the cap body is snap-fitted on the deck. For example, the first snap-fit portion may be a protruding structure, and correspondingly, the deck is provided on a proximal side thereof with an inclined surface that matches the protruding structure. Or the first snap-fit portion may be a hook-shaped structure, and correspondingly, the deck is provided on a proximal side thereof with a protruding structure that is snap-fitted with the hook-shaped structure.

Please refer to FIGs. 4 to 6, FIG. 4 shows a schematic structure diagram of the cap body of the present application in one embodiment, FIG. 5 shows a schematic structure diagram of the capsule chamber of the present application in one embodiment, and FIG. 6 shows a schematic structure diagram of the cap body and the capsule chamber of the present application in one embodiment. As shown in FIG. 4, the cap body is provided on a proximal side thereof with a first snap-fit portion 11, which is a protruding structure formed on an inner wall surface of the cap body at a proximal end thereof. As shown in FIG. 5, an inclined surface 45 is provided on a lower surface of the deck at a proximal end thereof. As shown in FIG. 6, when the cap body is closed, the first snap-fit portion 11 comes into contact with an edge of the deck, and when a force continues to be applied, the first snap-fit portion 11 moves downward and is snap-fitted on the inclined surface 45 of the deck. When the cap body needs to be opened, an upward force is applied to the cap body, and the first snap-fit portion 11 moves upward along the inclined surface 45 and is disengaged from the deck.

In the implementation, in order to facilitate the opening of the cap body during use, please refer to FIG. 7, which shows a schematic structure diagram of the cap body of the present application in another embodiment, the cap body is provided on a proximal side thereof with a first anti-skid portion 12. In the embodiment shown in FIG. 7, the first anti-skid portion may be a plurality of protruding or recessed structures formed on the proximal side of the cap body to form anti-skid structures. In other embodiments, the first anti-skid portion may also be a plurality of anti-skid structures or materials such as silicone strips arranged on the proximal side of the cap body.

In one exemplary embodiment, please refer further to FIG. 2, as shown in the figure, the mouthpiece body 21 comprises a sucking portion 211 and a mounting portion 212. The mounting portion 212 is configured to be snap-fitted on the deck of the capsule chamber, so that the mouthpiece is connected to the capsule chamber in a snap-fit manner, and the filter member can closely fit the capsule insertion port of the deck in a snap-fit state. As shown in FIG. 2, the sucking portion has an approximately flat elliptical structure adapted to the oral portion (mouth) of a human body, making it easy for a user to suck on an upper portion of the mouthpiece with his/her mouth, so that a negative pressure is formed in the inhalation channel of the mouthpiece by inhalation when the upper portion of the mouthpiece is wrapped in the mouth of the user.

The mounting portion 212 and the sucking portion 211 are integrally formed. During use, because the mounting portion matches the deck and the sucking portion matches the user's mouth, the mounting portion and the sucking portion have different shapes and are thus more ergonomic. For this reason, a transition surface is provided between the mounting portion 212 and the sucking portion 211 to achieve a transition from the approximately flat elliptical structure of the sucking portion 211 to the structure of the mounting portion that is shaped to match the deck.

In one exemplary embodiment, the mouthpiece comprises second snap-fit portions located on a proximal side thereof, the deck comprises third snap-fit portions located on a proximal side thereof, and the second snap-fit portions and the third snap-fit portions are snap-fitted at proximal ends thereof to achieve a snap fit between the deck and the mouthpiece.

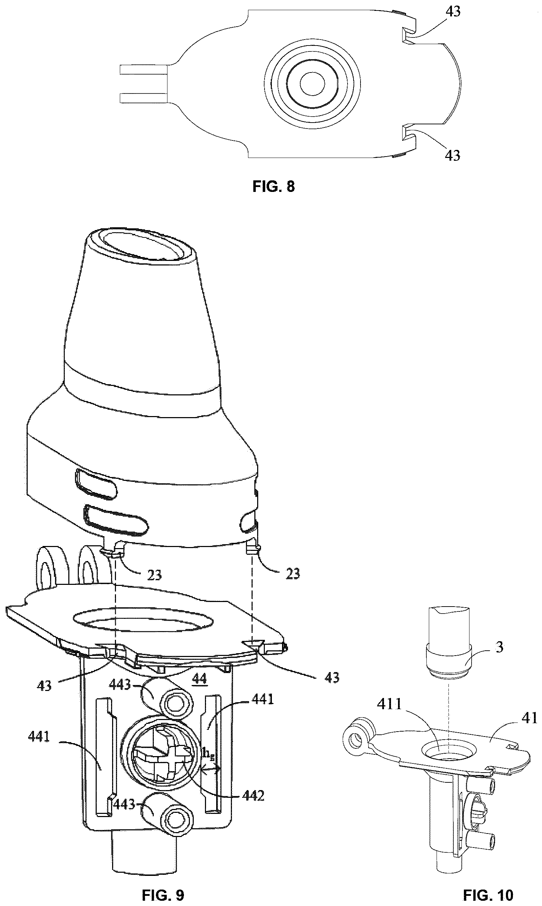

In a possible implementation, please refer further to FIG. 2 in conjunction with FIGs. 8 and 9. FIG. 8 shows a schematic diagram of the deck of the present application in one embodiment, and FIG. 9 shows a schematic diagram of the state in which the mouthpiece and the capsule chamber of the present application are snap-fitted in one embodiment. As shown in FIGs. 2 and 9, the second snap-fit portions 23 are hook structures formed on two sides of a lower surface of the mouthpiece, and as shown in FIGs. 8 and 9, the third snap-fit portions 43 are recesses located on two sides of the deck at the proximal end thereof and corresponding to the hook structures on the two sides of the mouthpiece, the recesses being notches formed on two sides of the deck. The hooks on the lower surface of the mouthpiece are slightly deformed under stress when contacting edges of the notches, and after the hooks are located below the notches, the hooks are restored from deformation so as to be snapped and fixed below the edges of the notches, thus realizing a snap fit between the mouthpiece and the capsule chamber. The snap-fit structure in the embodiment can make the filter member mounted at the bottom of the inhalation channel to completely fit the capsule insertion port of the deck, so that a powdered drug is completely inhaled together with the airflow to the lung of a patient by means of the inhalation of the patient.

In one implementation, please refer further to FIG. 2, in order to facilitate the operation of snap-fitting or disengaging the mouthpiece on or from the deck, second anti-skid portions 24 are provided on two sides of the proximal side of the mouthpiece. In the embodiment as shown in FIG. 2, the second anti-skid portions may be a plurality of protruding or recessed structures formed on the two sides of the proximal side of the mouthpiece to form anti-skid structures. In other embodiments, the second anti-skid portions may also be a plurality of anti-skid structures or materials such as silicone strips arranged on the proximal side of the mouthpiece.

In one exemplary embodiment, in order to facilitate the communication between the inhalation channel and the compartment, the bottom end of the inhalation channel extends out of the mouthpiece body. In the state in which the mouthpiece is snap-fitted on the deck, the filter member on the inhalation channel is press-fitted on the capsule insertion port. Further, contact surfaces of the filter member and the capsule chamber are provided with end surfaces that are shaped to match each other, so that in the state in which the mouthpiece is snap-fitted on the capsule chamber, the filter member can be press-fitted on and closely fit the capsule insertion port, to avoid airflow leakage during use.

In a possible implementation, please refer to FIG. 10, which shows a schematic structure diagram of the filter member and the capsule chamber of the present application in one embodiment. As shown in the figure, a stepped structure formed by the transition between the shape of the sleeving portion and the shape of the mesh portion is provided on an outer surface of a lower portion of the filter member, and correspondingly, a shape that correspondingly matches the stepped structure is provided on an upper portion of an inner surface of the capsule insertion port 411 of the deck, so that the filter member closely fits the capsule insertion port. When the mouthpiece is snap-fitted on the deck, the mesh portion is integrally located in the capsule insertion port 411. Therefore, when pharmaceutical powder is inhaled, airflow can better pass through the mesh portion under the action of a negative pressure to flow in an ideal flow direction, thereby ensuring effective inhalation of the pharmaceutical powder. In the embodiment, the capsule insertion port 411 on the deck is correspondingly engaged with the mesh portion 32 so that the mesh portion 32 closely fits the capsule insertion port 411 so as to ensure the inhalation efficiency.

In some embodiments, because the mouthpiece is snap-fitted on the third snap-fit portions of the deck by means of the second snap-fit portions, the filter member further closely fits the capsule insertion port, so that the airflow efficiency is higher during use, that is, the amount of pharmaceutical powder in the capsule inhaled can be guaranteed under a smaller inhalation force.

In one exemplary embodiment, a first annular sleeve-fit portion is formed on an outer surface of the bottom end of the inhalation channel, a second annular sleeve-fit portion is provided on an inner surface of the sleeving portion of the filter member, and the first annular sleeve-fit portion is configured to be snap-fitted on the second annular sleeve-fit portion.

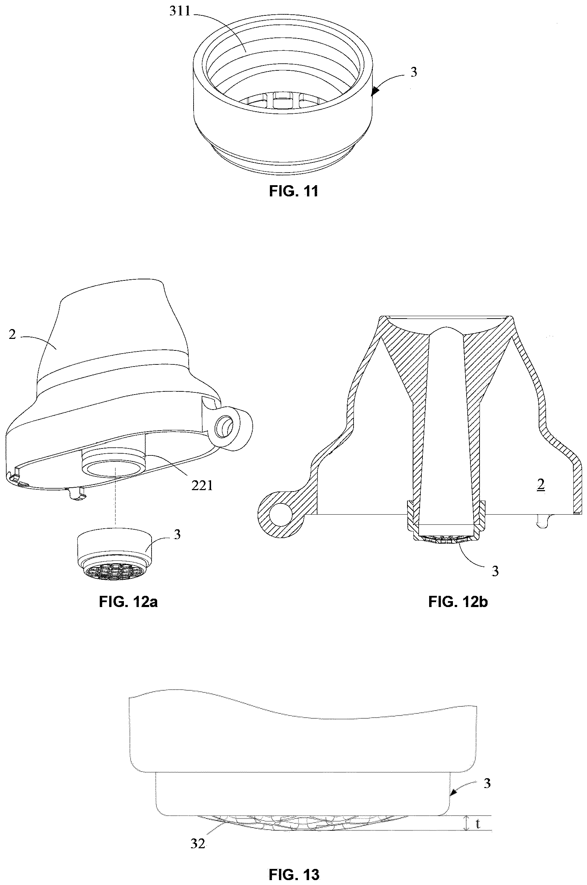

In a possible implementation, please refer to FIGs. 11, 12a and 12b in conjunction with FIG. 2. FIG. 11 shows a schematic structure diagram of the filter member of the present application in another embodiment, FIG. 12a shows a schematic structure diagram of the filter member and the mouthpiece of the present application in one embodiment, and FIG. 12b shows a cross-sectional view of the filter member and the mouthpiece of the present application in one embodiment. As shown in FIG. 2, a circle of raised rib 221 is provided on the inner surface of the sleeving portion of the filter member, and correspondingly, as shown in FIG. 11, an annular groove 311 matching the raised rib is provided on the outer surface of the bottom end of the inhalation channel, so that the raised rib 221 can be snapped and fixed into the annular groove 311 when the filter member 3 is mounted on the inhalation channel of the mouthpiece 2, as shown in FIG. 12a. FIG. 12b shows a schematic view of the cross-sectional structure after the filter member 3 is mounted on the inhalation channel.

It should be noted that although the structure of the raised rib matching the annular groove is taken as an example in the embodiment, it is not limited thereto in practical applications as long as an effect of snap-fit between the filter member and the inhalation channel can be achieved. For example, in other embodiments, the circle of raised rib in the above embodiment may be replaced with a plurality of bumps formed on the inner surface of the sleeving portion of the filter member, and the annular groove may be replaced with a plurality of holes formed in the outer surface of the inhalation channel and corresponding to the plurality of bumps on a one-to-one basis. In addition, the positions of the raised rib and the annular groove, or the positions of the bumps and the holes may also be interchanged. For example, the annular groove may be formed in the inner surface of the sleeving portion of the filter member and the circle of raised rib may be provided on the outer surface of the bottom end of the inhalation channel, or the plurality of holes may be formed in the inner surface of the sleeving portion of the filter member and the plurality of corresponding bumps may be provided on the outer surface of the bottom end of the inhalation channel, which will not be further elaborated here.

A fixing structure between the first annular sleeving portion and the second annular sleeving portion in the embodiment can make the filter member and the inhalation channel firmly engaged to ensure the use efficiency and safety.

In one exemplary embodiment, please refer to FIG. 13, which shows a schematic structure diagram of the filter member of the present application in yet another embodiment. As shown in the figure, in order to ensure the inhalation efficiency of pharmaceutical powder, the thickness t of the mesh portion 32 ranges from 0.35 to 0.51 mm. Here, the thickness comprises the distance between a lowest point and a highest point of the mesh portion. In addition, in order to ensure the inhalation efficiency while ensuring the filtration effect, a mesh count of the mesh portion should also be designed reasonably. It should be understood that the mesh count includes the number of meshes in the mesh portion and that the size of the meshes directly limits the diameter of an object that can pass through the mesh portion. Thus, the mesh count is related to the size of the object of the filter member and the airflow. In different embodiments, the mesh count is configured in the range of about 20 to 30, e.g. a value taken from 20, 21, 22, 23, 24, 25, 26, 27, 28, 29 and 30. In the embodiment, the thickness t of the mesh portion 32 ranges from 0.35 to 0.51 mm, the cross-sectional diameter of the filter member ranges from 11 to 12 mm, and the mesh count is 21.

The filter member in the present application can control the distribution of flowing airflow by means of the size and shape of the mesh and the surface curvature, and can also prevent a patient from inhaling a large fragmented capsule shell when vigorously inhaling pharmaceutical powder. In a possible implementation, a surface radius of curvature of the mesh portion may be from 13 to 14 mm, e.g. 13.1 mm, 13.2 mm, 13.3 mm, 13.4 mm, 13.5 mm, 13.6 mm, 13.7 mm, 13.8 mm, 13.9 mm, 14 mm, etc. In the embodiment as shown in FIG. 13, the surface radius of curvature of the mesh portion is 13.21 mm. It should be understood that curvature is used to describe the amount that a curve is curved, and radius of curvature can be understood as the radius of a circle having a size corresponding to a determined curvature.

In some embodiments, the integrally-formed filter member may be of a material having a polymer structure, that is, both the sleeving portion and the mesh portion are of a polymer material. The polymer material includes, but is not limited to, acrylonitrile butadiene styrene (ABS) plastic, polycarbonate (PC), polyamide (PA), etc. In the embodiment, the filter member is made of an ABS material to avoid deformation due to improper use. Moreover, the ABS material, a terpolymer of acrylonitrile (A), butadiene (B) and styrene (S), integrates the properties of the three components, acrylonitrile has high hardness and strength, heat resistance and corrosion resistance, butadiene has impact resistance and toughness, and styrene has high surface gloss and is easy to color and process, so that the filter member has high strength and good toughness and is low in cost and easy to process and form. In addition, the material having a polymer structure will not be deformed or rusted in a damp environment, thus ensuring the stability of the mesh structure.

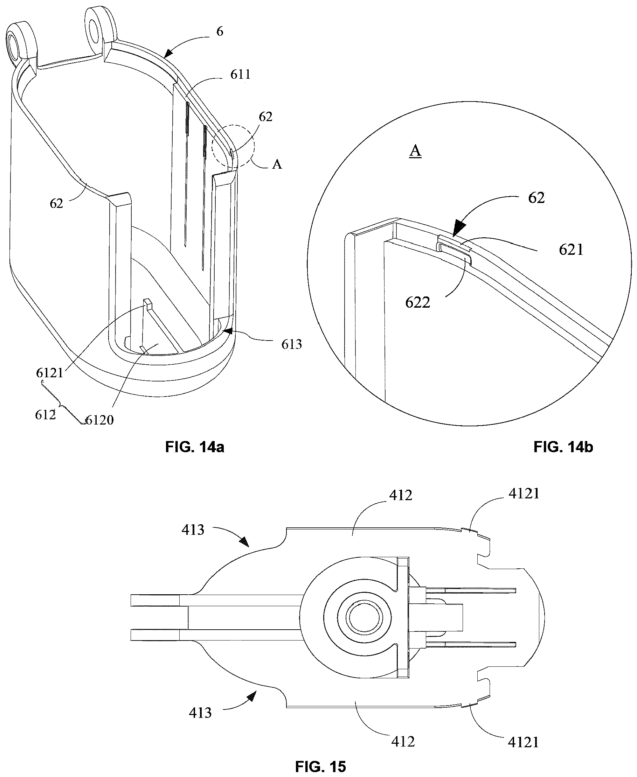

In one exemplary embodiment, please refer to FIG. 15, which shows a schematic structure diagram (bottom view) of the capsule chamber of the present application in one embodiment. As shown in the figure, the deck comprises side wings 412 located on two sides, and the side wings 412 are provided with fourth snap-fit portions 4121. Here, the fourth snap-fit portions are bumps located on two sides of the side wings. Please refer to FIG. 14a, which shows an enlarged partial structure diagram of the base of the present application in one embodiment, fifth snap-fit portions 62 corresponding to the fourth snap-fit portions 4121 are provided on upper edges of two sides of the base 6. Please refer to FIG. 14b, which shows an enlarged partial structure diagram of part A in FIG. 14a of the present application. Here, in order to clearly illustrate the structure of the fifth snap-fit portion, FIG. 14a is rotated at a certain angle and the position of the fifth snap-fit portion 62 is enlarged so as to obtain the state shown in FIG. 14b. As shown in the figure, the fifth snap-fit portion comprises an inclined surface 621 and a slot 622 located under the inclined surface 621. When the deck is snap-fitted, the fourth snap-fit portion 4121, which is a bump, comes into contact with the inclined surface 621 and then moves downward and is snap-fitted into the slot 622, thereby snap-fitting the capsule chamber on the base.

In one implementation, referring further to FIG. 14a, inward edgefolds 611 are provided on upper edges of two sides of the base body 61 of the base 6. When the deck is mounted on the base body, the side wings 412 on the two sides of the deck abut against the edgefolds 611, which can avoid the dislocation of the deck on one hand, and on the other hand provide support for front and back sides of the base, thus increasing the compressive strength of the base in front and back side directions.

In one exemplary embodiment, in order to form an airflow channel in the inhalation device, the capsule chamber needs an additional channel for communication with outside air in addition to communication with the inhalation channel of the mouthpiece, so that air can enter from the additional channel for communication with outside air after a user sucks on the mouthpiece with his/her mouth. Since the capsule chamber is snap-fitted on the base by means of the deck, the deck is provided with first air inlets. When a negative pressure is generated in the inhalation channel, outside air can easily enter an inner space of the base by means of the first air inlets, thereby providing outside air for the compartment of the capsule chamber.

In one implementation, in order to facilitate processing and molding, the first air inlets may be indentations formed in the contour of the deck. For example, referring further to FIG. 15, as shown in figure, the contour on two sides of the distal side of the deck is narrower than that of the side wings of the deck, thereby forming indentations 413. After the deck is snap-fitted on the base, the side wings are snap-fitted on an upper portion of the base body of the base, and a gap is provided between the base body of the base and the indentation 413 to serve as an entering channel for outside air. It should be noted herein that although the indentations formed on the two sides of the distal side of the deck are taken as an example in the embodiment, other structures may also be used in practical applications, as long as the first air inlets can be exposed after the mouthpiece is placed on the capsule chamber, so that airflow can enter from the first air Inlets during use.

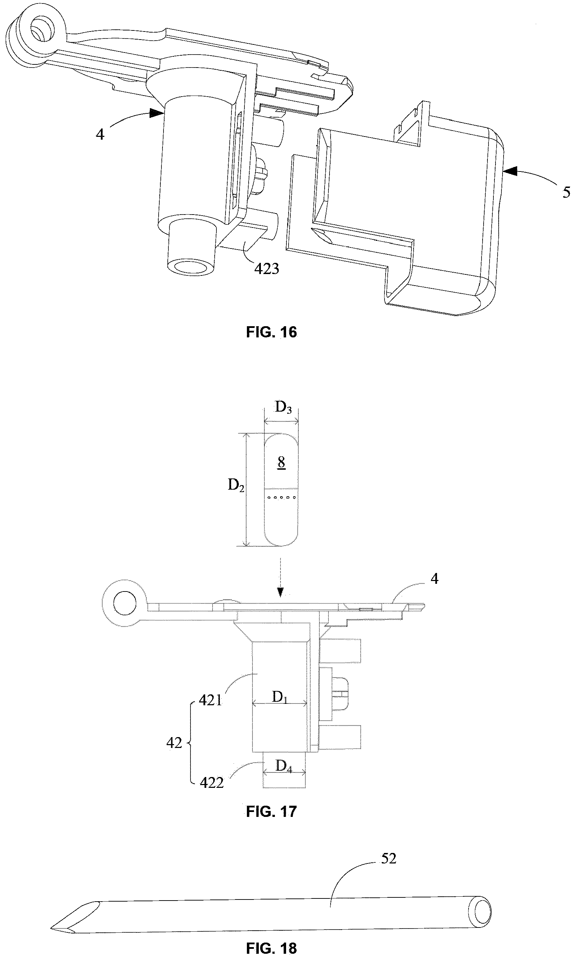

In one exemplary embodiment, the compartment 42 of the capsule chamber comprises a main compartment, which, in order to keep the capsule in an upright state in the main compartment so that the capsule is easily pierced by the piercing assembly, has a diameter greater than a transverse cross-sectional diameter of the capsule and smaller than a longitudinal cross-sectional diameter of the capsule. Please refer to FIG. 17, which shows a structural schematic diagram of the capsule chamber and the capsule of the present application in one embodiment. As shown in the figure, a transverse diameter D₁ of a main compartment 421 is larger than a transverse cross-sectional diameter D₃ of a capsule 8 so that the capsule 8 can enter the main compartment, and the transverse diameter D₁ of the main compartment 421 is smaller than a longitudinal cross-sectional diameter D₂ of the capsule 8 so that the capsule 8 is kept in an upright state in the main compartment 421. In a possible implementation, the ratio of the transverse diameter to the longitudinal diameter of the main compartment is 1: 2 to 1: 3.

It should be noted that in the embodiment, the size of the capsule is not limited, the capsule may be a capsule of any size, and the main compartment may also be configured to have a diameter that matches the size of the capsule in order to adapt to the size of the capsule. The size of the capsule is usually related to the dosage, that is, when the amount of powder is large, the capsule size is also designed to be large, and when the amount of powder is small, the capsule size is also designed to be small.

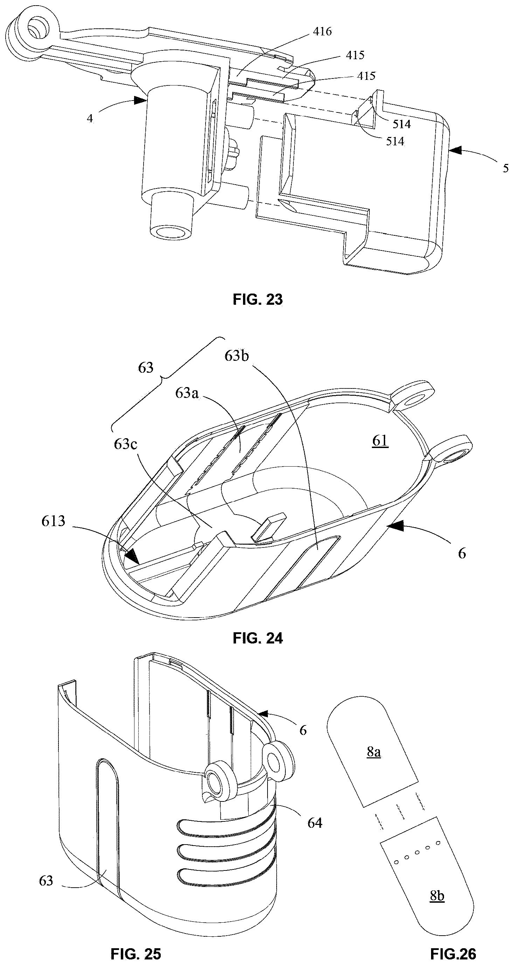

In one embodiment, taking into account the dosages of existing powdered drugs, such as tiotropium bromide and indanterol mentioned above, the transverse cross-sectional diameter D₃ of the capsule 8 is usually designed to be 5 to 6 mm, and the height, i.e., the longitudinal cross-sectional diameter D₂, is usually designed to be 15 to 16 mm. Moreover, in some embodiments, since a capsule is assembled from upper and lower shells, please refer to FIG. 26, which shows a schematic structure diagram of the capsule of the present application in one embodiment, a capsule shell is assembled from an upper shell 8a and a lower shell 8b, and the sum of longitudinal cross-sectional diameters of the upper shell 8a and the lower shell 8b is generally designed to be 16 to 17 mm.

In one exemplary embodiment, please refer further to FIG. 17, an auxiliary compartment 422 in communication with the main compartment 421 is provided at the bottom of the compartment 42 of the capsule chamber, and the auxiliary compartment 422 is configured to provide an air inlet channel for the main compartment 421. The auxiliary compartment 422 is provided at a bottom end thereof with a second air inlet, and a gap is formed between the second air inlet and the bottom surface of the base to enable the capsule chamber to be communicated with the inner space of the base body of the base to form an air inlet channel after being snap-fitted on the base body of the base. Furthermore, in order to prevent the capsule from falling from the main compartment to the auxiliary compartment, the diameter D₄ of the auxiliary compartment is smaller than the transverse cross-sectional diameter D₃ of the capsule.

In one exemplary embodiment, the compartment of the capsule chamber comprises a mounting structure that matches the piercing assembly so that the piercing assembly remains connected to the capsule chamber in a non-use state and is movable relative to the capsule chamber in a use state so as to puncture the capsule in the capsule chamber. The mounting structure comprises a mounting plate, and the mounting plate and the compartment may be of an integral structure. The mounting plate is provided with slots for enabling the button member of the piercing assembly to move relative to the capsule chamber without being disengaged from the capsule chamber, and tube portions configured to limit piercing directions of the piercing needles of the piercing assembly. In some embodiments, in order to apply a force to the piercing assembly during use so that the piercing assembly can automatically return to an initial position thereof after piercing the capsule, the piercing assembly further comprises a reset mechanism, an example of which comprises, but is not limited to, an elastic element such as a spring. In addition, the mounting plate is provided with an abutment portion at a position corresponding to the reset mechanism, so as to limit the position of the reset mechanism and ensure normal operation thereof. The positions at which the slots, the abutment portion and the tube portions are arranged may be determined according to the structure of the piercing assembly. For example, when the piercing assembly is movably connected to the mounting plate by means of the left and right sides of the button member, the piercing assembly pierces the capsule in the capsule chamber by means of two piercing needles arranged one on top of the other, and the reset mechanism is arranged between the two piercing needles, the slots may be correspondingly formed in symmetrical left and right sides of the mounting plate, the abutment portion may be located between the symmetrical slots, and the two tube portions corresponding to the two piercing needles may be respectively located on upper and lower sides of the abutment portion.

In one exemplary embodiment, the capsule chamber is further provided, at a position facing the button member, with a first stop portion, which is configured to limit a movement position of the piercing assembly relative to the capsule chamber. The first stop portion may be located on the main compartment and may also be located on the auxiliary compartment, and the first stop portion is configured to have a length that matches an ideal travel range of the button member, that is, in the process of pressing the button member, the button member stops traveling when touching the first stop portion, and at the moment, the piercing needles can just pierce the capsule without excessive puncture. Therefore, when the first stop portion is located on the auxiliary compartment, the length of the first stop portion located on the auxiliary compartment should be slightly larger than the length of the first stop portion located on the main compartment, since the transverse diameter of the auxiliary compartment is smaller than that of the main compartment. In an embodiment where a capsule chamber comprises a mounting plate, the first stop portion may also be arranged on the mounting plate.

In one embodiment, please refer to FIG. 16, which is a schematic structure diagram of the piercing assembly and the capsule chamber of the present application in yet another embodiment, as shown in the figure, a first stop portion 423 is arranged on a side facing the button member at a lower portion of the main compartment, and the first stop portion 423 is located on the mounting plate. When the button member is pressed, the button member cannot continue to move toward the distal end after the bottom of the button member contacts the first stop portion 423, thereby avoiding excessive puncture.

In one implementation, there may be one or more piercing needles in the piercing assembly, which are fixedly connected at proximal ends thereof to the button member, and the positions and number of tube portions on the mounting plate correspond to the positions and number of piercing needles. After the piercing assembly is mounted in the capsule chamber, the piercing needles can be passed through the corresponding tube portions on the mounting plate, i.e. the distal sides of the piercing needles are located in the corresponding tube portions. When a force is applied to the button member, the piercing needles can move along the tube portions so as to pierce the capsule in the compartment after extending into the compartment.

The piercing needle is a component configured to pierce a capsule, and the piercing needle is not necessarily of an elongated structure, but may also be of a thin sheet structure. For example, the piercing needle may also be designed in a flat shape similar to a blade structure, and correspondingly, the tube portion on the mounting plate may be adjusted to be of a flat tube structure, so that the blade-shaped piercing needle can move in the tube thereof. In this embodiment, when the piercing needles pierce the capsule, the capsule is transversely cut so that the capsule has a large opening, or the capsule is directly cut into two or more pieces.

In a possible implementation, in order to ensure that the piercing needles can function and improve the drug escape efficiency, the distance between the two needles farthest from each other should be less than the height of the capsule and greater than half of the height of the capsule. For example, in the case where there are two piercing needles, a distance between the two piercing needles should be less than a height of the capsule and greater than half of the height of the capsule. For example, when the piercing needles pierce the capsule, the position at which the wall of the capsule is broken is just located at the transition position between a main body portion and a top portion on two ends of the capsule. In the embodiment, the distance between holes formed in the capsule after the piercing needles pierce the capsule is 3/5 to 4/5 of the height of the assembled capsule.

The manner in which the piercing needles are arranged may be configured according to the direction in which the capsule is placed and the number of piercing needles. For example, in the case where there is one piercing needle, the piercing needle may be arranged at a position corresponding to the center of the capsule; in the case where there are two piercing needles and the capsule is placed vertically, the two piercing needles can be arranged one on top of the other; in the case where there are two piercing needles and the capsule is placed transversely, the two piercing needles can be arranged left and right; and in the case where there are three or more piercing needles, the piercing needles may or may not be on the same straight line. It should be understood that the positions at which the piercing needles are arranged are examples only and are not limiting, and may be adjusted according to specific requirements in practical applications, which will not be further elaborated herein.

In one exemplary embodiment, please refer to FIG. 18, which shows a schematic structure diagram of the piercing needle of the present application in one embodiment, as shown in the figure, in order to facilitate the piercing of the capsule by the piercing needle and to minimize the generation of capsule fragments, a needle tip portion of the piercing needle 52 has an inclined surface, and the piercing needle is designed to be eccentric in position, i.e. the tip point of the needle tip is not on the same straight line as the axis position of the piercing needle. When the capsule shell is pierced, pieces of the capsule at the breach are not liable to fall off from the capsule, which fundamentally avoids the generation of capsule fragments to ensure the safety and comfort in the inhalation process. Meanwhile, the structure can also make a powdered drug be effectively released during inhalation.

In one implementation, in order to facilitate the movable connection of the piercing assembly to the mounting plate, the button member of the piercing assembly comprises a pressing portion and movable connecting portions. The pressing portion and the movable connecting portions are of an integrated structure, where a limiting portion matching one of the symmetrical slots is provided at a distal end of each of the movable connecting portions, so that the piercing assembly can move relative to the capsule chamber without being disengaged from the mounting plate.

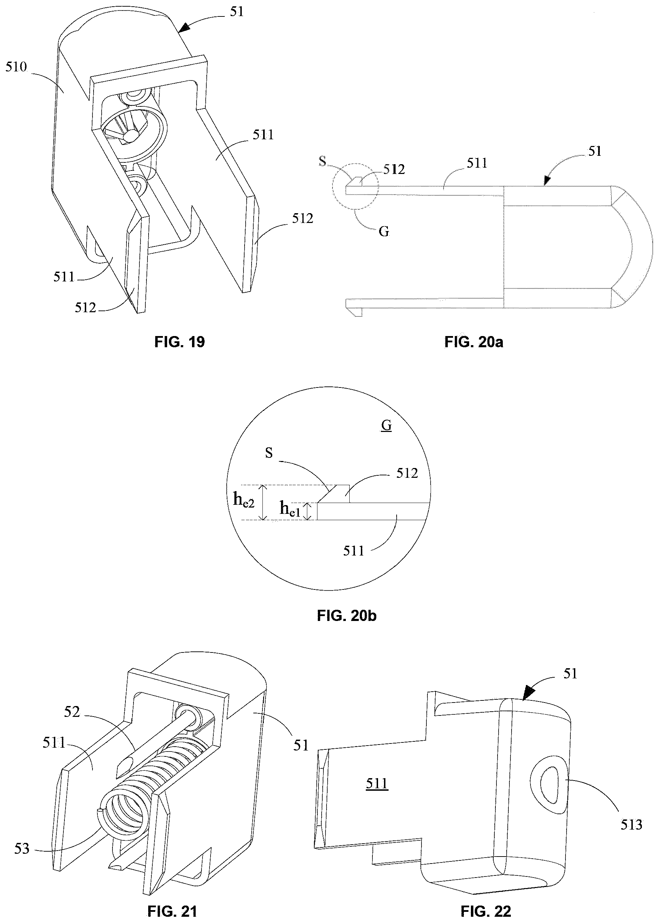

In a possible implementation, please refer to FIG. 19, which shows a structural diagram of the button member of the present application in one embodiment, as shown in FIG. 19, a button member 51 comprises a pressing portion 510 and movable connecting portions, which are integrally formed. In the embodiment, the movable connecting portions are extension arms 511 on two sides of the button member 51 that extend distally, each of the extension arms 511 is provided at a distal end thereof with a protruding hook portion 512 as a limiting portion, and the hook portion and the extension arm are of an integrated structure.

Correspondingly, please refer further to FIG. 9, the mounting plate 44 is provided with two slots 441, the positions of which correspond to the positions of the extension arms 511, and the width of the slots 441 is slightly larger than the width of the extension arms 511, so that the extension arms 511 can slide in the slots. Meanwhile, the sum of the width of the hook portion 512 and the width of the extension arm 511 is larger than the width of the slot 441, so that the extension arm does not disengage from the mounting plate when sliding in the slot 441.

In some cases, please refer to FIG. 20a, which shows a schematic structure diagram of the button member of the present application in another embodiment, and FIG. 20b, which shows an enlarged schematic view of part G in FIG. 20a, and in conjunction with FIG. 9, as shown in the figures, in order to further facilitate the assembly of the piercing assembly 5 to the mounting plate 44, the hook portion 512 has an inclined surface s, the spacing between the inclined surface s and the extension arm 511 increases from distal to proximal sides, and the minimum width hₑ₁ at the inclined surface is smaller than the width h_{g} of the slot 441, and the maximum width hₑ₂ at the inclined surface is slightly larger than the width h_{g} of the slot 441. Here, as shown in FIG. 20b, the minimum width hₑ₁ at the inclined surface comprises the sum of widths of a distal side of the hook and a distal side of the extension arm, and the maximum width hₑ₂ at the inclined surface comprises the sum of widths of a proximal side of the hook and a proximal side of the extension arm. On the basis of this structural design, during mounting, the distal side of the extension arm 511 is aligned with the slot 441 on the mounting plate 44. Since the minimum width hₑ₁ at the inclined surface is smaller than the width h_{g} of the slot 441, the extension arm 511 can pass through the slot, and with the increase in width at the inclined surface, the inclined surface gradually contacts the edge of the slot 441 closely. After force application continues, the slot 441 applies a pressure to the inclined surface, and the extension arm produces a slight elastic deformation under stress. Finally, when the whole hook portion 512 passes through the slot 441, the inclined surface returns to an initial state and the piercing assembly is limited by the hook portion 512, so that the piercing assembly can move relative to the capsule chamber without being disengaged from the mounting plate.

It should be noted that the structure of the limiting portion in the embodiment is only an example but not limiting. In practical applications, the limiting portion may also be of other structures as long as the button member can pass through the mounting plate and be limited thereon. For example, the hook portions in FIGs. 20a and 20b may also be replaced with a plurality of small hooks, as limiting portions, distributed on the distal sides of the extension arms.

Please refer to FIG. 21, which shows a schematic structure diagram of the piercing assembly of the present application in yet another embodiment, and in conjunction with FIG. 9. In the embodiment, please refer to FIG. 21 first, there are two piercing needles 52 which are arranged one on top of the other, and the two piercing needles 52 are both located between the front and rear extension arms 511 and are fixed to the button member 51 by means of proximal sides. Please refer to FIG. 9, the mounting plate 44 of the capsule chamber is provided with two tube portions 443 corresponding to the two piercing needles so that the two tube portions 443 are also arranged one on top of the other on the mounting plate 44, and the tube portions 443 are in communication with the compartment 42 of the capsule chamber 4. After the piercing assembly 5 is mounted in the capsule chamber, the piercing needles are arranged in the tube portions 443 in a penetrating manner, i.e. the distal sides of the piercing needles 52 are located in the corresponding tube portions 443. When a force is applied to the button member 51, the piercing needles 52 can move along the tube portions 443 so as to pierce the capsule in the compartment after extending into the compartment 42. The penetrating means that the diameter of the piercing needle is smaller than the diameter of the tube portion so that the piercing needle can pass through the hollow portion of the tube portion. Moreover, to facilitate the movement of the piercing needle along the tube portion, a portion of the piercing needle, e.g. the distal end of the piercing needle, may be located in the tube portion in an initial state (i.e. in a state in which the button member is not operated by applying a force).

In order to apply a force to the piercing assembly during use so that the piercing assembly can automatically return to an initial position thereof after piercing the capsule, the piercing assembly further comprises a reset mechanism 53, an example of which comprises, but is not limited to, a spring, etc. Moreover, the mounting plate 44 is provided with an abutment portion 442 at a position corresponding to the reset mechanism 53, so as to achieve a function of limiting the position of the reset mechanism and ensuring normal operation thereof. Herein, the abutment portion 442 may be a mounting base for fixing the reset mechanism 53. For example, in the case where the reset mechanism is a spring, the abutment portion 442 may be a spring mounting base projecting proximally so that a distal side of the spring may be sleeved on the mounting base. In the embodiment as shown in FIG. 9, in consideration of the space utilization efficiency of the mounting plate, the abutment portion 442 is arranged at the center of the mounting plate, that is, between the left and right slots 441 and between the upper and lower tube portions 443. In other embodiments, the reset mechanism may also be arranged at other positions as long as the button member can be reset after being stressed. The structure of the abutment portion may also be designed according to actual requirements and is not limited to a spring mounting base protruding proximally. For example, the abutment portion may also be a recessed portion formed in the surface of the mounting plate so that the reset mechanism is mounted therein.

It should be noted that in the above embodiment, the capsule chamber is designed to be of a vertical structure so that the capsule is placed in an upright state in the capsule chamber. In other possible embodiments, the capsule chamber may also be designed to be of a transverse structure so that the capsule is placed in the capsule chamber in a transverse state, and accordingly, the piercing needles in the piercing assembly and the tube portions on the mounting plate are arranged horizontally to match the direction in which the capsule is placed, so as to pierce the capsule.

In one implementation, please refer further to FIG. 22, which is a schematic structure diagram of the button member of the present application in one embodiment, as shown in the figure, in order to facilitate the pressing of a button portion, a third anti-skid portion 513 is provided on an outer surface of the pressing portion, so that the action force can be more effectively transferred when a force is applied by means of the button member 51. Given that a user usually presses the button member with his/her thumb during use, the third anti-skid portion is designed as a recessed structure with an arc-shaped outer edge surface as shown in FIG. 22 to adapt to the finger shape of a human body. It should be understood that the third anti-skid portion in the embodiment is merely an example instead of limiting. In practical applications, the third anti-skid portion may also be a plurality of protruding or recessed structures formed on the proximal side of the cap body to form an anti-skid structure. In other embodiments, the third anti-skid portion may also be a plurality of anti-skid structures or materials such as silicone strips arranged on the proximal side of the cap body.

In one exemplary embodiment, in order to further ensure the motion stability of the piercing needles when the piercing assembly pierces the capsule, corresponding guide structures are also provided on the deck and the button member. The guide structures are structures for making the button member move in a straight line under force, so as to drive the piercing needles to move more stably, thereby avoiding the deviation of the piercing needles during piercing or the generation of a sense of resistance caused by the unstable movement position of the button member during pressing.

In one exemplary embodiment, the capsule chamber is further provided, at a position facing the button member, with a third stop portion, which is configured to limit a movement position of the piercing assembly relative to the capsule chamber. The third stop portion may be located on the main compartment and may also be located on the auxiliary compartment, the third stop portion is configured to have a length that matches an ideal travel range of the button member, that is, in the process of pressing the button member, the button member stops traveling when touching the third stop portion, and at the moment, the piercing needles can just pierce the capsule without excessive puncture. Therefore, when the third stop portion is located on the auxiliary compartment, the length of the third stop portion located on the auxiliary compartment should be slightly larger than the length of the third stop portion located on the main compartment, since the transverse diameter of the auxiliary compartment is smaller than that of the main compartment. In an embodiment where a capsule chamber comprises a mounting plate, the third stop portion may also be arranged on the mounting plate.

In one embodiment, please refer to FIG. 16, which is a schematic structure diagram of the piercing assembly and the capsule chamber of the present application in yet another embodiment, as shown in the figure, a third stop portion 423 is arranged on a side facing the button member at a lower portion of the main compartment, and the third stop portion 423 is located on the mounting plate. When the button member is pressed, the button member cannot continue to move toward the distal end after the bottom of the button member contacts the third stop portion 423, thereby avoiding excessive puncture.

When the piercing needles pierce a capsule (or break the wall of the capsule), the consistency and stability of the piercing directions are particularly important for the inhalation device. On the basis of this, in the embodiments of the present application, a first guide portion is provided on a lower surface of the side of the deck corresponding to the button member, i.e., a proximal side of the deck, and the button member is provided with a second guide portion matching the first guide portion, so that when the button member moves towards the capsule chamber, in the process of pushing a button to pierce a capsule, the two needles can be guided to move stably on a horizontal line so as to ensure that holes in a capsule shell are kept relatively consistent in shape and size after the capsule is pierced by the needles. Therefore, not only can pharmaceutical powder in the capsule chamber escape smoothly under the action of a negative pressure, but also in the process of piercing the capsule, fragments of capsule skin will not be produced due to the shaking of the needles.

The first guide portion and the second guide portion may comprise the form in which a lead rail matches a lead groove, and may also comprise the form in which a limiting groove matches a guide block, etc. For example, the first guide portion may comprise a lead rail formed on a proximal lower surface of the deck, and the second guide portion comprises a lead groove provided in an upper surface of the button member, or the second guide portion comprises a lead rail formed on the proximal lower surface of the deck, and the first guide portion comprises a lead groove provided in the upper surface of the button member.

In one implementation, please refer to FIG. 23, which shows a schematic structure diagram of the piercing assembly and the capsule chamber of the present application in one embodiment. As shown in the figure, two elongated lead rails 415 are arranged on the proximal lower surface of the deck, and correspondingly, an upward extension arm is arranged on an upper side of the button member, and the extension arm is provided with lead grooves 514 at positions corresponding to the lead rails 415. When a force is applied to the button member, the lead grooves 514 on the button member can move along the lead rails 415 of the deck, thereby better controlling the movement direction of the button member to drive the piercing needles to pierce the capsule in the capsule chamber more accurately and efficiently. In another embodiment, the positions of the lead rails and the lead grooves may also be interchanged, that is, the lead rails may be located on the button member while the lead grooves may be located on the lower surface of the deck, which can also achieve the same technical effect.

In another implementation, limiting blocks may be arranged on two sides of the proximal lower surface of the deck, the movable connecting portions of the button member may be extended upward to contact the deck, and the movable connecting portions on two sides of the button member are located between and abut against the two limiting blocks on the deck, so that when a force is applied to the button member, the movable connecting portions of the button member can be limited by the limiting blocks to move stably, so as to drive the piercing needles to pierce the capsule in the capsule chamber more accurately and efficiently.

It should be noted that the structures of the first guide portion and the second guide portion in the above embodiment are merely examples instead of being limiting. In practical applications, it only needs to ensure that they function to guide the button member so as to maintain stable movement thereof. In addition, the positions at which the first guide portion and the second guide portion are arranged may be adjusted according to specific requirements, which will not be further described herein.

In one exemplary embodiment, to avoid excessive puncture of the capsule when the button member is pressed, the deck further comprises a third stop portion, which is a stop structure located on the deck and configured to limit the range of movement of the button member relative to the deck, and includes, but is not limited to a stopper, a baffle, etc. In the embodiment, please refer further to FIG. 23. As shown in the figure, the third stop portion is a bump 416 formed at the distal end of the first guide portion, so that when the button member moves along the deck, the button member is limited and cannot continue to advance after abutting against the bump, thus limiting a movement position of the piercing assembly relative to the capsule chamber.

In one embodiment, the base body of the base may also be provided with a second stop portion, which is a stop structure located on the base and configured to limit the range of movement of the button member relative to the base, and includes, but is not limited to a stopper, a baffle, etc. In the embodiment, please refer further to FIG. 14a, a baffle 612 is provided below a proximal side of the base body of the base, and the baffle 612 is provided with an upwardly protruding stopper 6121 on a distal side thereof. In this case, the part of the baffle other than the stopper is defined as a baffle body 6120. The height of the baffle body 6120 is lower than the lower surface of the button member, thus allowing the button member to pass, while the height of the stopper 6121 is higher than the lower surface of the button member, thus not allowing the button member to pass. Therefore, when the button member is pressed, the button member may first move towards the capsule chamber along the top surface of the baffle body 6120, and the button member cannot continue to move towards the capsule chamber when touching the baffle 6121, so that the depth at which the piercing needles pierce the capsule can be controlled by limiting a movement position of the piercing assembly relative to the capsule chamber.

In one exemplary embodiment, the capsule chamber is further provided, at a position facing the button member, with a first stop portion, which is configured to limit a movement position of the piercing assembly relative to the capsule chamber. The first stop portion may be located on the main compartment and may also be located on the auxiliary compartment, the first stop portion is configured to have a length that matches an ideal travel range of the button member, that is, in the process of pressing the button member, the button member stops traveling when touching the first stop portion, and at the moment, the piercing needles can just pierce the capsule without excessive puncture. Therefore, when the first stop portion is located on the auxiliary compartment, the length of the first stop portion located on the auxiliary compartment should be slightly larger than the length of the first stop portion located on the main compartment, since the transverse diameter of the auxiliary compartment is smaller than that of the main compartment. In an embodiment where a capsule chamber comprises a mounting plate, the first stop portion may also be arranged on the mounting plate.

In one embodiment, please refer to FIG. 16, which is a schematic structure diagram of the piercing assembly and the capsule chamber of the present application in yet another embodiment, as shown in the figure, a first stop portion 423 is arranged on a side facing the button member at a lower portion of the main compartment, and the first stop portion 423 is located on the mounting plate. When the button member is pressed, the button member cannot continue to move toward the distal end after the bottom of the button member contacts the first stop portion 423, thereby avoiding excessive puncture. In one exemplary embodiment, please refer further to FIG. 14a, as shown in the figure, the base is provided at a proximal end thereof with an opening 613, so that after the capsule chamber is assembled into the base, a mounting space and a movement space are provided for the piercing assembly by means of the opening 613, and the piercing assembly can move along the capsule chamber under a stressed state.

In one exemplary embodiment, in order to facilitate the observation of the state of the capsule in the capsule chamber during operation, the base further comprises a transparent inspection window mounted on the base body, and the compartment of the capsule chamber is also made of a transparent material, so that the state of the capsule in the chamber and the state in which the wall of the capsule is broken during penetration can be observed by means of the transparent inspection window and the transparent compartment during use.

The position at which the transparent inspection window is arranged on the base may be designed according to actual requirements, for example, the transparent inspection window may be arranged on front and rear sides of the base.

In one implementation, please refer to FIG. 24, which shows a schematic structure diagram of the base in another embodiment, as shown in the figure, in order to facilitate the mounting of the transparent inspection window and the base, the transparent inspection window 63 can be configured to be of a U-shaped structure, and correspondingly, the base body 61 of the base is provided on a bottom surface thereof with a mounting groove extending to front and rear sides of the base body, two long arms 63a, 63b of the U-shaped structure correspond to the mounting grooves in front and rear portions of the base body 61 respectively, and a bottom plate 63c of the U-shaped structure corresponds to the mounting groove in the bottom surface of the base body 61, so that the transparent inspection window of the U-shaped structure is mounted on the base 6. In the embodiment, the transparent inspection window 63 of the U-shaped structure is mounted on the base 6 by means of hot-melting or bonding; or the transparent inspection window 63 of the U-shaped structure may be integrally formed with the base 6.

In other embodiments, the base may also be made of a transparent material or a partially transparent material (e.g. an inspection window portion is made of a transparent material), so that the state of the capsule in the compartment and the state in which the wall the capsule is broken upon piercing can be observed by means of the transparent base and the transparent compartment.

In one exemplary embodiment, please refer to FIG. 25, which shows a schematic structure diagram of the base of the present application in yet another embodiment, in order to facilitate holding during use, the base is provided on a distal side thereof with a fourth anti-skid portion 64. The fourth anti-skid portion may be a plurality of protruding or recessed structures formed on the proximal side of the cap body so as to meet the ergonomics or the holding habit of users, and may also be a plurality of anti-skid structures such as silicone strips arranged on the distal side of the base. Further, the base is configured to be of an approximately cuboid structure, making it easier to hold in use, so that a user feels more comfortable when applying a force.

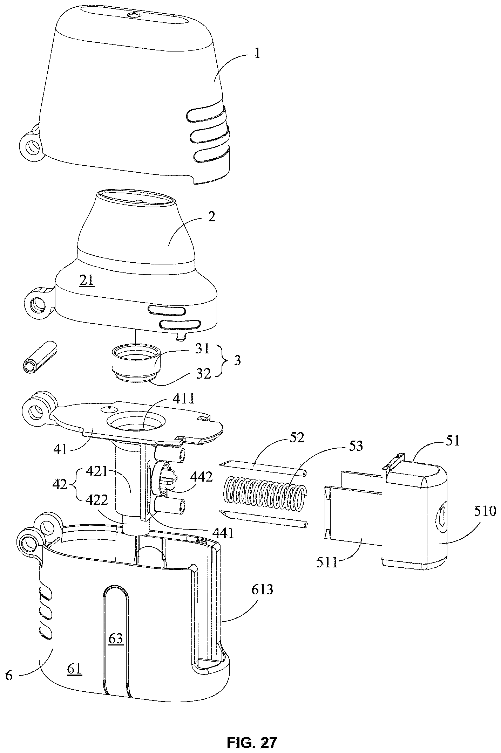

In one exemplary embodiment, please refer to FIG. 27, which shows a schematic structure diagram of the inhalation device of the present application in yet another embodiment. As shown in the figure, the inhalation device comprises a cap body 1, a mouthpiece 2, a filter member 3, a capsule chamber 4, a piercing assembly 5, and a base 6. The cap body 1, the mouthpiece 2, the capsule chamber 4 and the base 6 are coaxially hinged on distal sides thereof. In order to facilitate the use by an operator, anti-skid portions are respectively provided on a proximal side of the cap body, a proximal side of the mouthpiece, an outer surface of a pressing portion of a button member and a distal side of a base body.

Corresponding snap-fit structures are arranged on proximal sides of the cap body 1 and a deck 41, so that the cap body can be opened or closed relative to the base when the base and the deck are snap-fitted. An upper portion of a mouthpiece body 21 of the mouthpiece 2 is configured to match the mouth of a human body so that a user inhales a powdered drug into the respiratory tract by means of the mouthpiece. The bottom of an inhalation channel of the mouthpiece 2 is of a tubular structure, and the filter member 3 is sleeved on the tubular structure. A sleeving portion 31 of the filter member 3 matches a groove in an outer surface of a lower portion of the inhalation channel 22 by means of a circle of raised rib on an inner surface of the sleeving portion 31, so that the filter member 3 is snapped and fixed at the bottom of the inhalation channel. In addition, the sleeving portion 31 and a mesh portion 32 of the filter member are of an integrated structure, so that manufacture and production are facilitated while filtering and fixing functions are achieved, and the step of assembling the mesh portion and the sleeving portion is omitted.

The capsule chamber 4 is located below the mouthpiece 2. The capsule chamber comprises a deck 41 and a compartment 42 that are integrated, and the compartment 42 includes a main compartment 421 and an auxiliary compartment 422. A capsule can be placed into the compartment via a capsule insertion port in the deck. Because the diameter of the main compartment is larger than the transverse cross-sectional diameter of the capsule and smaller than the longitudinal cross-sectional diameter of the capsule, and the diameter of the auxiliary compartment is smaller than the transverse cross-sectional diameter of the capsule, the capsule is kept in an upright state in the main compartment, making it easier to be pierced by the piercing assembly. An upper portion of the auxiliary compartment is in communication with the main compartment, a lower portion thereof is also provided with an opening, and a gap is provided between the auxiliary compartment and the inner bottom of the base, so that outside airflow can enter the capsule chamber. Corresponding snap-fit structures are arranged on proximal sides of the mouthpiece 2 and the deck 41, so that the mouthpiece can be snapped and fixed to the deck. Notches are provided on two sides of a distal side of the deck, so that an air inlet is provided after the deck is mounted on the base. The capsule insertion port and the filter member are provided with end surfaces that are shaped to match each other, so that in the state in which the mouthpiece is snap-fitted on the capsule chamber, the filter member can be press-fitted on and closely fit the capsule insertion port, to avoid air leakage.

The piercing assembly 5 maintains a positional relationship with the capsule chamber 4 by means of the mounting plate 44. The piercing assembly 5 comprises a button member 51, two piercing needles 52 and a reset mechanism 53. The button member 51 comprises a pressing portion 510 and movable connecting portions 511 located on two sides of a distal end of the pressing portion, the mounting plate is provided with slots 441 at positions corresponding to the movable connecting portions, and the limiting portions on the movable connecting portions can limit the button member in the slots so that the button member can move along the slots without being disengaged from the slots. The reset mechanism is located between the movable connecting portions on the two sides, and an abutment portion 442 is arranged at a position on the mounting plate that corresponds to the reset mechanism. One side of the reset mechanism 53 is connected to the button member 51, and the other side thereof is mounted on the abutment portion 442, so as to help the button member return to an initial position thereof after pressing the button member. The two piercing needles are respectively fixed on the button member at proximal ends thereof and are arranged one on top of the other, and correspondingly, the mounting plate is provided with tube portions respectively at positions corresponding to the two piercing needles, so that after the piercing assembly is mounted on the mounting plate of the capsule chamber, the two piercing needles are respectively located in the corresponding tube portions. Because the tube portions are in communication with the main compartment, when the button member is pressed, the button member can drive the piercing needles to move to the main compartment through the tube portions to pierce the capsule.

Herein, in order to better control the movement direction of the button member so as to drive the piercing needles to pierce the capsule in the capsule chamber more accurately and efficiently, two elongated lead rails are arranged on a proximal lower surface of the deck, and correspondingly, an upward extension arm is provided on an upper side of the button member, and lead grooves are formed at positions on the extension arm that correspond to the lead rails. When a force is applied to the button member, the lead grooves in the button member can move along the lead rails of the deck to avoid the deviation of the piercing needles during piercing or the generation of a sense of resistance caused by the unstable movement position of the button member during pressing. More importantly, because of the guide design of the button member, in the process of pushing a button to pierce the capsule, the two needles can be guided to move stably on a horizontal line, so as to ensure that holes in a capsule shell are kept relatively consistent in shape and size after the capsule is pierced by the needles. Therefore, not only can pharmaceutical powder in the capsule chamber escape smoothly under the action of a negative pressure, but also in the process of piercing the capsule, fragments of capsule skin will not be produced due to the shaking of the needles.

Further, to avoid excessive puncture of the capsule when the button member is pressed, a third stop portion is further provided at a distal end of the lead rail. When the button member moves along the deck, the button member is limited and cannot continue to advance after abutting against the bump, thus limiting a movement position of the piercing assembly relative to the capsule chamber. Meanwhile, a baffle is provided below a proximal side in the base body of the base, and the baffle is provided with an upwardly protruding stopper on a distal side thereof. The height of a baffle body is lower than the lower surface of the button member, thus allowing the button member to pass, while the height of the stopper is higher than the lower surface of the button member, thus not allowing the button member to pass. Therefore, when the button member is pressed, the button member may first move towards the capsule chamber along a top surface of the baffle body, and the button member cannot continue to move towards the capsule chamber when touching the baffle, so that the depth at which the piercing needles pierce the capsule can be controlled by limiting the movement position of the piercing assembly relative to the capsule chamber.

The base 6 comprises a base body 61, which has a hollow structure inside and is configured to accommodate the compartment 42 of the capsule chamber. Corresponding snap-fit structures are provided on two sides of the deck 41 of the capsule chamber 4 and two sides of an upper portion of the base body 61, so that the capsule chamber can be disassembled from the base. The base body 61 is provided on a proximal side thereof with an opening 613, so that after the capsule chamber is assembled into the base, a movement space is provided for the piercing assembly by means of the opening 613, and the piercing assembly can move along the capsule chamber under a stressed state.

The base further comprises a U-shaped transparent inspection window 63, a mounting groove extending to front and back sides of the base body is provided in a bottom surface of the base body 61 of the base, the transparent inspection window 63 is mounted on the base body 61, and the compartment of the capsule chamber is also made of a transparent material, so that the state of the capsule in the compartment can be observed by means of the transparent inspection window and the transparent compartment during use.

During use, first, the cap body 1 and the mouthpiece 2 are opened, a capsule is placed into the main compartment 421 via the capsule insertion port 411 in the deck 41, and then, the mouthpiece 2 and the cap body 1 are closed, and the button member is pressed so that the button member drives the piercing needles to enter the main compartment along the tube portions to pierce the capsule. At the moment, on one hand, the pressing process is smoother under the action of the lead rails and the lead grooves between the deck and the button member, and on the other hand, excessive puncture by the piercing assembly is avoided under the action of the third stop portions on the lead rail and the second stop portions on the base. When a user stops the force application to the button member, the reset mechanism resets the button member to an initial position thereof.

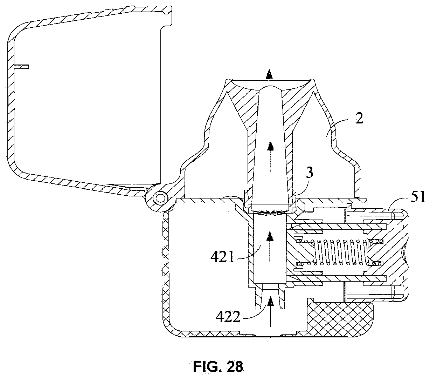

Then, the user opens the cap body and sucks on the upper portion of the mouthpiece with his/her mouth to inhale. Please refer to FIG. 28, which shows a schematic diagram of the airflow direction of the inhalation device of the present application in one embodiment. As shown in the figure, under a negative pressure generated in the mouthpiece 2, outside airflow enters the base via a gap (i.e., a first air inlet) between the deck and the base, and enters the main compartment 421 via the bottom of the auxiliary compartment 422. Due to the structural design of the mesh portion, airflow favorable for escape of powder is generated in the main compartment, and the powder enters the inhalation channel of the mouthpiece 2 via the mesh portion, while capsule fragments are blocked outside the mesh portion, and the powder in the inhalation channel is thus inhaled into the body of the user.

Finally, the mouthpiece is opened to clear away the capsule shell, fragments, etc. in the capsule chamber, and/or after washing and drying, the mouthpiece and the cap body are mounted in place for later use.

The inhalation device in the present application uses the filter member and the capsule chamber which are of integral design, so that the difficulty and cost of production are reduced while the use effect is ensured. Moreover, in the inhalation device of the present application, the filter member closely fits the capsule insertion port, and when the mouthpiece is snap-fitted on the deck, the mesh portion is integrally located in the capsule chamber. Therefore, when pharmaceutical powder is inhaled, airflow can better pass through the mesh portion under the action of a negative pressure to flow in an ideal flow direction, thereby ensuring effective inhalation of the pharmaceutical powder to ensure the amount of pharmaceutical powder in the capsule inhaled under a smaller inhalation force. Matching guide structures of the deck and the button member can avoid the deviation of the piercing needles during piercing or the generation of a sense of resistance caused by the unstable movement position of the button member during pressing. The cap body, the mouthpiece, the filter member and the base of the inhalation device are coaxially connected and anti-skid structures are provided at a plurality of positions, which conform to the ergonomic design.

On the basis of the above examples, the present application provides the following embodiments:
1. An inhalation device, comprising:
   a mouthpiece, which comprises a mouthpiece body and an inhalation channel formed in the mouthpiece body, wherein the bottom of the inhalation channel is of a tubular structure;
   a filter member, which comprises a sleeving portion sleeved on a bottom end of the inhalation channel and a mesh portion integrally formed with the sleeving portion to avoid that capsule fragments generated after a capsule is pierced enter the inhalation channel, wherein the sleeving portion and the mesh portion are both made of a polymer material;
   a capsule chamber, which is located below the mouthpiece and comprises a deck and a compartment integrally formed on a lower side of the deck and configured to hold a capsule in a use state, wherein the deck is provided with a capsule insertion port which is in communication with the compartment and is correspondingly engaged with the mesh portion;
   a piercing assembly, which is arranged on one side of the capsule chamber and comprises a button member and piercing needles which are movably arranged on one side of the capsule chamber, wherein the button member, when stressed in a use state, moves relative to the capsule chamber to drive the piercing needles to pierce the capsule in the capsule chamber;
   a base, which comprises a base body for accommodating the compartment and part of the piercing assembly; and
   a cap body, which covers the mouthpiece when in a state of being snap-fitted on the base;
   wherein in a state in which the capsule is in the capsule chamber and the capsule is pierced, when a negative pressure is generated in the inhalation channel, pharmaceutical powder in the capsule enters the inhalation channel via the mesh portion through the capsule insertion port.
2. The inhalation device according to embodiment 1, wherein a first annular sleeving portion is formed on an outer surface of a bottom end of the inhalation channel, and a second annular sleeving portion configured to be snap-fitted on the first annular sleeving portion is provided on an inner surface of the sleeving portion of the filter member.
3. The inhalation device according to embodiment 1, wherein a surface radius of curvature of the mesh portion ranges from 13 to 14 mm.
4. The inhalation device according to embodiment 1, wherein a mesh count of the mesh portion ranges from 20 to 30.
5. The inhalation device according to embodiment 1, wherein a thickness of the mesh portion ranges from 0.35 to 0.51 mm.
6. The inhalation device according to embodiment 1, wherein the mouthpiece body comprises:
   a mounting portion configured to be snap-fitted on the deck of the capsule chamber; and
   a sucking portion integrally formed with the mounting portion and having an approximately flat elliptical structure adapted to the mouth of a human body;
   wherein a transition surface is provided between the mounting portion and the sucking portion.
7. The inhalation device according to embodiment 1, wherein the mouthpiece comprises second snap-fit portions located on a proximal side of the mouthpiece, and the deck comprises third snap-fit portions located on a proximal side of the deck, the second snap-fit portions and the third snap-fit portions being snap-fitted at proximal ends thereof.
8. The inhalation device according to embodiment 7, wherein the second snap-fit portions are hook structures formed on a lower surface of the mouthpiece, and the third snap-fit portions are recesses formed on the deck and corresponding to the hook structures.
9. The inhalation device according to embodiment 1, wherein the mouthpiece comprises a second anti-skid portion located on the proximal side of the mouthpiece.
10. The inhalation device according to embodiment 1, wherein a bottom end of the inhalation channel extends out of the mouthpiece body, so that when the mouthpiece is snap-fitted on the deck, the filter member sleeved on the inhalation channel is press-fitted on the capsule insertion port, so as to form a closed channel of airflow.
11. The inhalation device according to embodiment 1 or 10, wherein contact surfaces of the filter member and the capsule chamber are provided with matching end surfaces so that the filter member closely fits the capsule chamber when the mouthpiece is snap-fitted on the capsule chamber.
12. The inhalation device according to embodiment 1, wherein the cap body, the mouthpiece, the capsule chamber and the base are coaxially hinged on distal sides thereof by means of hinge portions.
13. The inhalation device according to embodiment 1, wherein the cap body comprises a first snap-fit portion located on a proximal side of the cap body and configured to be snap-fitted on an edge of a proximal side of the deck.
14. The inhalation device according to embodiment 13, wherein the first snap-fit portion is a bump located on an inner side of a proximal end of the cap body, and the deck is provided at a proximal end thereof with an end surface that matches the bump, so that the cap body and the deck are snap-fitted on proximal sides thereof.
15. The inhalation device according to embodiment 1, wherein the cap body comprises a first anti-skid portion located on the proximal side of the cap body.
16. The inhalation device according to embodiment 1, wherein the deck comprises side wings provided with fourth snap-fit portions, which are configured to be snap-fitted on fifth snap-fit portions on an edge of an upper side of the base.
17. The inhalation device according to embodiment 1, wherein the deck is provided with a first air inlet, which is configured to facilitate the entry of outside air into an inner space of the base via the first air inlet when a negative pressure is generated in the inhalation channel.
18. The inhalation device according to embodiment 1, wherein the compartment of the capsule chamber comprises a main compartment having a diameter greater than a transverse cross-sectional diameter of the capsule and smaller than a longitudinal cross-sectional diameter of the capsule.
19. The inhalation device according to embodiment 18, wherein an auxiliary compartment in communication with the main compartment is provided at the bottom of the compartment of the capsule chamber, the auxiliary compartment is provided with a second air inlet at a bottom end thereof, a gap is disposed between the second air inlet and a bottom surface of the base, and the auxiliary compartment has a diameter smaller than the transverse cross-sectional diameter of the capsule.
20. The inhalation device according to embodiment 1, wherein the compartment of the capsule chamber comprises a mounting structure, which is configured to match the piercing assembly so that the piercing assembly pierces the capsule in the capsule chamber by using the piercing needles thereof during movement relative to the capsule chamber.
21. The inhalation device according to embodiment 20, wherein the mounting structure comprises:
   a mounting plate provided with symmetrical slots;
   an abutment portion formed on the mounting plate and located between the symmetrical slots; and
   two tube portions formed on the mounting plate, respectively located on upper and lower sides of the abutment portion, in communication with the compartment and configured to limit piercing directions of the piercing needles.
22. The inhalation device according to embodiment 21, wherein the piercing assembly further comprises a reset mechanism located between the button member and the abutment portion and configured to be pressed, when the button member moves toward the capsule chamber, to provide a reset force to restore the piercing needles and the button member to initial positions thereof.
23. The inhalation device according to embodiment 21, wherein the piercing needles are arranged in the tube portions in a penetrating manner, and proximal ends of the piercing needles are fixedly connected to the button member.
24. The inhalation device according to embodiment 21, wherein the button member comprises a pressing portion and movable connecting portions integrally formed with the pressing portion, wherein a limiting portion matching one of the symmetrical slots is provided at a distal end of each of the movable connecting portions.
25. The inhalation device according to embodiment 24, wherein a third anti-skid portion is provided on an outer surface of the pressing portion.
26. The inhalation device according to embodiment 21, wherein the capsule chamber is provided, towards a proximal side, with a first stop portion, which is configured to limit a movement position of the piercing assembly relative to the capsule chamber.
27. The inhalation device according to embodiment 1, wherein a needle tip portion of each of the piercing needles has an inclined surface.
28. The inhalation device according to embodiment 1, wherein two piercing needles are provided, the two piercing needles are arranged one on top of the other, and a distance between the two piercing needles arranged one on top of the other is less than a height of the capsule and greater than one half of the height of the capsule.
29. The inhalation device according to embodiment 1, wherein the base body of the base comprises a fourth anti-skid portion located on a distal side of the base body.
30. The inhalation device according to embodiment 1, wherein the base is provided, at a proximal end thereof, with an opening, which is configured to provide a movement space for the piercing assembly.
31. The inhalation device according to embodiment 1, wherein the base body of the base is provided with a second stop portion, which is configured to limit a movement position of the piercing assembly relative to the capsule chamber.
32. The inhalation device according to embodiment 1, wherein the base further comprises a transparent inspection window mounted on the base body, and the compartment of the capsule chamber has a transparent wall surface, so as to observe the state of the capsule in the capsule chamber through the base in a use state.
33. The inhalation device according to embodiment 32, wherein the transparent inspection window is U-shaped, and correspondingly, a mounting groove extending to front and back sides of the base body is formed in a bottom surface of the base body of the base, so as to mount the transparent inspection window.
34. The inhalation device according to embodiment 1, wherein the base is made of a transparent material, and the compartment of the capsule chamber is provided with a transparent wall surface, so as to observe the state of the capsule in the capsule chamber through the base in a use state.
35. An inhalation device, comprising:
   a mouthpiece, which comprises a mouthpiece body and an inhalation channel formed in the mouthpiece body;
   a filter member, which is sleeved on a bottom end of the inhalation channel and is configured to avoid that capsule fragments generated after a capsule is pierced enter the inhalation channel;
   a capsule chamber, which is located below the mouthpiece and comprises a deck and a compartment located on a lower side of the deck and configured to hold a capsule in a use state, wherein the deck is provided with a capsule insertion port which is in communication with the compartment and is correspondingly engaged with the filter member, and a first guide portion is provided on a proximal side of a lower surface of the deck;
   a piercing assembly, which is arranged on one side of the capsule chamber and comprises a button member and piercing needles which are movably arranged on one side of the capsule chamber, wherein the button member is provided with a second guide portion matching the first guide portion, and the button member, when stressed in a use state, moves linearly relative to the capsule chamber to drive the piercing needles to pierce the capsule in the capsule chamber;
   a base, which comprises a base body for accommodating the compartment and part of the piercing assembly; and
   a cap body, which covers the mouthpiece when in a state of being snap-fitted on the base;
   wherein in a state in which the capsule is in the capsule chamber and the capsule is pierced, when a negative pressure is generated in the inhalation channel, pharmaceutical powder in the capsule enters the inhalation channel via the filter member through the capsule insertion port.
36. The inhalation device according to embodiment 35, wherein the first guide portion is a lead rail, and the second guide portion is a lead groove matching the lead rail; or the first guide portion is a lead groove, and the second guide portion is a lead rail matching the lead groove.
37. The inhalation device according to embodiment 35, wherein the first guide portion is provided at a distal end thereof with a third stop portion, which is configured to limit a movement position of the piercing assembly relative to the capsule chamber.
38. The inhalation device according to embodiment 35, wherein the base body of the base is provided with a second stop portion, which is configured to limit a movement position of the piercing assembly relative to the capsule chamber.
39. The inhalation device according to embodiment 35, wherein the cap body, the mouthpiece, the capsule chamber and the base are coaxially hinged on distal sides thereof by means of hinge portions.
40. The inhalation device according to embodiment 35, wherein the cap body comprises a first snap-fit portion located on a proximal side of the cap body and configured to be snap-fitted on an edge of a proximal side of the deck.
41. The inhalation device according to embodiment 40, wherein the first snap-fit portion is a bump located on an inner side of a proximal end of the cap body, and the deck is provided at a proximal end thereof with an end surface that matches the bump, so that the cap body and the deck are snap-fitted on proximal sides thereof.
42. The inhalation device according to embodiment 35, wherein the cap body comprises a first anti-skid portion located on the proximal side of the cap body.
43. The inhalation device according to embodiment 35, wherein the mouthpiece body comprises:
   a mounting portion configured to be snap-fitted on the deck of the capsule chamber; and
   a sucking portion integrally formed with the mounting portion and having an approximately flat elliptical structure adapted to the mouth of a human body;
   wherein a transition surface is provided between the mounting portion and the sucking portion.
44. The inhalation device according to embodiment 35, wherein the mouthpiece comprises second snap-fit portions located on a proximal side of the mouthpiece, and the deck comprises third snap-fit portions located on a proximal side of the deck, the second snap-fit portions and the third snap-fit portions being snap-fitted at proximal ends thereof.
45. The inhalation device according to embodiment 44, wherein the second snap-fit portions are hook structures formed on a lower surface of the mouthpiece, and the third snap-fit portions are recesses formed on the deck and corresponding to the hook structures.
46. The inhalation device according to embodiment 35, wherein the mouthpiece comprises a second anti-skid portion located on the proximal side of the mouthpiece.
47. The inhalation device according to embodiment 35, wherein a bottom end of the inhalation channel extends out of the mouthpiece body, so that when the mouthpiece is snap-fitted on the deck, the filter member sleeved on the inhalation channel is press-fitted on the capsule insertion port, so as to form a closed channel of airflow.
48. The inhalation device according to embodiment 35 or 47, wherein contact surfaces of the filter member and the capsule chamber are provided with matching end surfaces so that the filter member closely fits the capsule chamber when the mouthpiece is snap-fitted on the capsule chamber.
49. The inhalation device according to embodiment 35, wherein the deck comprises side wings provided with fourth snap-fit portions, which are configured to be snap-fitted on fifth snap-fit portions on an edge of an upper side of the base.
50. The inhalation device according to embodiment 35, wherein the deck is provided with a first air inlet, which is configured to facilitate the entry of outside air into an inner space of the base via the first air inlet when a negative pressure is generated in the inhalation channel.
51. The inhalation device according to embodiment 35, wherein the compartment of the capsule chamber comprises a main compartment having a diameter greater than a transverse cross-sectional diameter of the capsule and smaller than a longitudinal cross-sectional diameter of the capsule.
52. The inhalation device according to embodiment 51, wherein an auxiliary compartment in communication with the main compartment is provided at the bottom of the compartment of the capsule chamber, the auxiliary compartment is provided with a second air inlet at a bottom end thereof, a gap is disposed between the second air inlet and a bottom surface of the base, and the auxiliary compartment has a diameter smaller than the transverse cross-sectional diameter of the capsule.
53. The inhalation device according to embodiment 35, wherein the compartment of the capsule chamber comprises a mounting structure, which is configured to match the piercing assembly so that the piercing assembly pierces the capsule in the capsule chamber by using the piercing needles thereof during movement relative to the capsule chamber.
54. The inhalation device according to embodiment 53, wherein the mounting structure comprises:
   a mounting plate provided with symmetrical slots;
   an abutment portion formed on the mounting plate and located between the symmetrical slots; and
   two tube portions formed on the mounting plate, respectively located on upper and lower sides of the abutment portion, in communication with the compartment and configured to limit piercing directions of the piercing needles.
55. The inhalation device according to embodiment 54, wherein the piercing assembly further comprises a reset mechanism located between the button member and the abutment portion and configured to be pressed, when the button member moves toward the capsule chamber, to provide a reset force to restore the piercing needles and the button member to initial positions thereof.
56. The inhalation device according to embodiment 54, wherein the piercing needles are arranged in the tube portions in a penetrating manner, and proximal ends of the piercing needles are fixedly connected to the button member.
57. The inhalation device according to embodiment 54, wherein the button member comprises a pressing portion and movable connecting portions integrally formed with the pressing portion, wherein a limiting portion matching one of the symmetrical slots is provided at a distal end of each of the movable connecting portions.
58. The inhalation device according to embodiment 57, wherein a third anti-skid portion is provided on an outer surface of the pressing portion.
59. The inhalation device according to embodiment 35 or 54, wherein the capsule chamber is provided, towards a proximal side, with a first stop portion, which is configured to limit a movement position of the piercing assembly relative to the capsule chamber.
60. The inhalation device according to embodiment 35, wherein a needle tip portion of each of the piercing needles has an inclined surface.
61. The inhalation device according to embodiment 35, wherein two piercing needles are provided, the two piercing needles are arranged one on top of the other, and a distance between the two piercing needles arranged one on top of the other is less than a height of the capsule and greater than one half of the height of the capsule.
62. The inhalation device according to embodiment 35, wherein the base body of the base comprises a fourth anti-skid portion located on a distal side of the base body.
63. The inhalation device according to embodiment 35, wherein the base is provided, at a proximal end thereof, with an opening, which is configured to provide a movement space for the piercing assembly.
64. The inhalation device according to embodiment 35, wherein the base further comprises a transparent inspection window mounted on the base body, and the compartment of the capsule chamber has a transparent wall surface, so as to observe the state of the capsule in the capsule chamber through the base in a use state.
65. The inhalation device according to embodiment 64, wherein the transparent inspection window is U-shaped, and correspondingly, a mounting groove extending to front and back sides of the base body is formed in a bottom surface of the base body of the base, so as to mount the transparent inspection window.
66. The inhalation device according to embodiment 35, wherein the base is made of a transparent material, and the compartment of the capsule chamber is provided with a transparent wall surface, so as to observe the state of the capsule in the capsule chamber through the base in a use state.

The above examples are merely illustrative of the principle and efficacy of the application and are not intended to limit the application. Any skilled person can modify or change the aforementioned examples without departing from the spirit and scope of the present application. Therefore, all equivalent modifications or changes made by those of ordinary skills in the art to which the application belongs without departing from the spirit and technical ideas disclosed in the application shall still be covered by the appended claims of the application.

## Claims

1. An inhalation device, comprising:
a mouthpiece, which comprises a mouthpiece body and an inhalation channel formed in the mouthpiece body, wherein the bottom of the inhalation channel is of a tubular structure;
a filter member, which comprises a sleeving portion sleeved on a bottom end of the inhalation channel and a mesh portion integrally formed with the sleeving portion to avoid that capsule fragments generated after a capsule is pierced enter the inhalation channel;
a capsule chamber, which is located below the mouthpiece and comprises a deck and a compartment integrally formed on a lower side of the deck and configured to hold a capsule in a use state, wherein the deck is provided with a capsule insertion port which is in communication with the compartment and is correspondingly engaged with the mesh portion;
a piercing assembly, which is arranged on one side of the capsule chamber and comprises a button member and piercing needles which are movably arranged on one side of the capsule chamber, wherein the button member, when stressed in a use state, moves relative to the capsule chamber to drive the piercing needles to pierce the capsule in the capsule chamber;
a base, which comprises a base body for accommodating the compartment and part of the piercing assembly; and
a cap body, which covers the mouthpiece when in a state of being snap-fitted on the base;
wherein in a state in which the capsule is in the capsule chamber and the capsule is pierced, when a negative pressure is generated in the inhalation channel, pharmaceutical powder in the capsule enters the inhalation channel via the mesh portion through the capsule insertion port.

2. The inhalation device according to claim 1, wherein the cap body, the mouthpiece, the capsule chamber and the base are coaxially hinged on distal sides thereof by means of hinge portions.

3. The inhalation device according to claim 1, wherein the cap body comprises a first snap-fit portion located on a proximal side of the cap body and configured to be snap-fitted on an edge of a proximal side of the deck.

4. The inhalation device according to claim 3, wherein the first snap-fit portion is a bump located on an inner side of a proximal end of the cap body, and the deck is provided at a proximal end thereof with an end surface that matches the bump, so that the cap body and the deck are snap-fitted on proximal sides thereof.

5. The inhalation device according to claim 1, wherein the cap body comprises a first anti-skid portion located on a proximal side of the cap body.

6. The inhalation device according to claim 1, wherein the mouthpiece body comprises:
a mounting portion configured to be snap-fitted on the deck of the capsule chamber; and
a sucking portion integrally formed with the mounting portion and having an approximately flat elliptical structure adapted to the mouth of a human body;
wherein a transition surface is provided between the mounting portion and the sucking portion.

7. The inhalation device according to claim 1, wherein the mouthpiece comprises second snap-fit portions located on a proximal side of the mouthpiece, and the deck comprises third snap-fit portions located on a proximal side of the deck, the second snap-fit portions and the third snap-fit portions being snap-fitted at proximal ends thereof.

8. The inhalation device according to claim 7, wherein the second snap-fit portions are hook structures formed on a lower surface of the mouthpiece, and the third snap-fit portions are recesses formed on the deck and corresponding to the hook structures.

9. The inhalation device according to claim 1, wherein the mouthpiece comprises a second anti-skid portion located on a proximal side of the mouthpiece.

10. The inhalation device according to claim 1, wherein the bottom end of the inhalation channel extends out of the mouthpiece body, so that when the mouthpiece is snap-fitted on the deck, the filter member sleeved on the inhalation channel presses the capsule insertion port, so as to form a closed channel of airflow.

11. The inhalation device according to claim 1 or 10, wherein contact surfaces of the filter member and the capsule chamber are provided with matching end surfaces so that the filter member closely fits the capsule chamber when the mouthpiece is snap-fitted on the capsule chamber.

12. The inhalation device according to claim 1, wherein the deck comprises side wings provided with fourth snap-fit portions, which are configured to be snap-fitted on fifth snap-fit portions on an edge of an upper side of the base.

13. The inhalation device according to claim 1, wherein the deck is provided with a first air inlet, which is configured to facilitate the entry of outside air into an inner space of the base via the first air inlet when a negative pressure is generated in the inhalation channel.

14. The inhalation device according to claim 1, wherein the compartment of the capsule chamber comprises a main compartment having a diameter greater than a transverse cross-sectional diameter of the capsule and smaller than a longitudinal cross-sectional diameter of the capsule.

15. The inhalation device according to claim 14, wherein an auxiliary compartment in communication with the main compartment is provided at the bottom of the compartment of the capsule chamber, wherein the auxiliary compartment is provided with a second air inlet at a bottom end thereof, wherein a gap is disposed between the second air inlet and a bottom surface of the base, and the auxiliary compartment has a diameter smaller than the transverse cross-sectional diameter of the capsule.

16. The inhalation device according to claim 1, wherein the capsule chamber is further provided, at a position facing the button member, with a first stop portion, which is configured to limit a movement position of the piercing assembly relative to the capsule chamber.

17. The inhalation device according to claim 1, wherein the compartment of the capsule chamber comprises a mounting structure, which is configured to match the piercing assembly so that the piercing assembly pierces the capsule in the capsule chamber by using the piercing needles thereof during movement relative to the capsule chamber.

18. The inhalation device according to claim 17, wherein the mounting structure comprises:
a mounting plate provided with symmetrical slots;
an abutment portion formed on the mounting plate and located between the symmetrical slots; and
two tube portions formed on the mounting plate, respectively located on upper and lower sides of the abutment portion, in communication with the compartment and configured to limit piercing directions of the piercing needles.

19. The inhalation device according to claim 18, wherein the piercing assembly further comprises a reset mechanism located between the button member and the abutment portion and configured to be pressed, when the button member moves toward the capsule chamber, to provide a reset force to restore the piercing needles and the button member to initial positions thereof.

20. The inhalation device according to claim 18, wherein the piercing needles are arranged in the tube portions in a penetrating manner, and proximal ends of the piercing needles are fixedly connected to the button member.

21. The inhalation device according to claim 18, wherein the button member comprises a pressing portion and movable connecting portions integrally formed with the pressing portion, wherein a limiting portion matching one of the symmetrical slots is provided at a distal end of each of the movable connecting portions.

22. The inhalation device according to claim 21, wherein a third anti-skid portion is provided on an outer surface of the pressing portion.

23. The inhalation device according to claim 1, wherein a needle tip portion of each of the piercing needles has an inclined surface.

24. The inhalation device according to claim 1, wherein two piercing needles are provided, wherein the two piercing needles are arranged one on top of the other, and a distance between the two piercing needles arranged one on top of the other is less than a height of the capsule and greater than one half of the height of the capsule.

25. The inhalation device according to claim 1, wherein the base body of the base comprises a fourth anti-skid portion located on a distal side of the base body.

26. The inhalation device according to claim 1, wherein the base is provided, at a proximal end thereof, with an opening, which is configured to provide a movement space for the piercing assembly.

27. The inhalation device according to claim 1, wherein the base body of the base is provided with a second stop portion, which is configured to limit a movement position of the piercing assembly relative to the capsule chamber.

28. The inhalation device according to claim 1, wherein the base further comprises a transparent inspection window mounted on the base body, and the compartment of the capsule chamber has a transparent wall surface, so as to observe the state of the capsule in the capsule chamber through the base in a use state.

29. The inhalation device according to claim 28, wherein the transparent inspection window is U-shaped, and correspondingly, a mounting groove extending to front and back sides of the base body is formed in a bottom surface of the base body of the base, so as to mount the transparent inspection window.

30. The inhalation device according to claim 1, wherein the base is made of a transparent material, and the compartment of the capsule chamber is provided with a transparent wall surface, so as to observe the state of the capsule in the capsule chamber through the base in a use state.

31. The inhalation device according to claim 1, wherein the sleeving portion and the mesh portion are both made of a polymer material.

32. The inhalation device according to claim 1, wherein a first annular sleeve-fit portion is formed on an outer surface of a bottom end of the inhalation channel, and a second annular sleeve-fit portion configured to be snap-fitted on the first annular sleeve-fit portion is provided on an inner surface of the sleeving portion of the filter member.

33. The inhalation device according to claim 1, wherein a surface radius of curvature of the mesh portion ranges from 13 to 14 mm.

34. The inhalation device according to claim 1, wherein a mesh count of the mesh portion ranges from 20 to 30.

35. The inhalation device according to claim 1, wherein a thickness of the mesh portion ranges from 0.35 to 0.51 mm.

36. The inhalation device according to claim 1, wherein a first guide portion is provided on a proximal side of a lower surface of the deck, and the button member is provided with a second guide portion matching the first guide portion.

37. The inhalation device according to claim 36, wherein the first guide portion is a lead rail, and the second guide portion is a lead groove matching the lead rail; or the first guide portion is a lead groove, and the second guide portion is a lead rail matching the lead groove.

38. The inhalation device according to claim 36, wherein the first guide portion is provided at a distal end thereof with a third stop portion, which is configured to limit a movement position of the piercing assembly relative to the capsule chamber.
